# EUROPEAN PATENT APPLICATION

(11) **EP 3 967 674 A1**
(43) Date of publication of application: **16.03.2022**
(21) Application number: 20306019.9
(22) Date of filing: 11.09.2020
(51) Int. Cl.: C07B 59/00

(54) **REACTIONS OF RADIOACTIVE COMPOUNDS FACILITATED BY A SOLID PHASE**

(71) Applicant: Precirix N.V., 1090 Brussels (BE)
(72) Inventor: Navarro, Laurent, 1000 BRUSSELS (BE); Verbrugge, Nicolas, 1200 Woluwe St. L., BRUSSELS (BE); D`Huyvetter, Matthias, 2018 ANTWERP (BE); Friebe, Matthias, 8142 UITIKON (CH); Tadino, Vincent, 5650 CHASTRÈS (BE); Maindron, Nicolas, 69200 VENISSIEUX (FR); Joyard, Yoann, 69003 LYON (FR)
(74) Representative: Nederlandsch Octrooibureau

(57) **Abstract**

The current invention provides a method for performing chemical reactions of radioactive compounds, and a device, system and method for improved heating for chemical reactions.

## Description

### Field

The invention relates to the field of reactions of radioactive compounds facilitated by a solid phase, and heating devices and systems for performing such reactions.

### Background of the invention

The synthesis of radioactive products typically comprises a labeling reaction of a non-radioactive compound to form a radioactive compound called a precursor, followed by a post-labeling reaction of said precursor to form a radioactive product. Many post-labeling reactions, especially but not limited to the hydrolytic removal of protecting groups, are characterized by a high activation barrier. Nevertheless, such reactions are typically carried out at room temperature, or at slightly elevated temperatures such as 20°C to 30°C, since higher temperatures may lead to an increase in radiolysis and/or unwanted side reactions. Therefore, these post-labeling reactions with a high activation barrier are performed in the art with long reaction times and/or under harsh reaction conditions. Mosdzianowski et al. (2002) [1], for example, shows that the use of a temperature of 60°C leads to an unwanted epimerization product during a hydrolysis catalyzed by a strong base (12 N NaOH), even if a solid phase is used.

Hence, there is an unmet need in the art for a method to perform a post-labeling reaction of a radioactive compound, specifically a hydrolysis, with a short reaction time, without significant radiolysis and with a good yield.

Furthermore, although such post-labeling reactions require controlled heating, they are typically performed in disposable reaction vessels. There is thus a need for controlled heating suitable to be used with any type of container, including disposable containers.

US8476063B2 [2] discloses devices and methods involving chambers heated by means of heating elements contained in fixtures. However, heating according to US8476063B2 lacks accuracy and temperature control and is overly complex.

US20020183660A1 [3], US9408257B2 [4] and KR101320762B1 [5] provide heating means but none of them is suitable for use with chemical reaction vessels. Moreover, these devices are overly complex and/or do not allow adequate temperature control.

### Description of the invention

### Method for performing a post-labeling chemical reaction

### General aspects

In a first aspect, the invention provides a method for performing a chemical reaction of a radioactive compound comprised in a mixture, wherein said method comprises the following steps: (a) contacting said mixture with a solid phase followed by (b) heating said mixture to a temperature selected in the range from 30°C up to 150°C, wherein steps (a) and (b) do not involve contacting said solid phase with an alkaline solution, and wherein said chemical reaction does not result in the formation of a new bond on a radionuclide comprised in said radioactive compound. Such methods are referred to in the current application as methods according to or of the invention. In the context of this application, said radioactive compound does not comprise fluorine-18. A (chemical) reaction according to or of the invention is a chemical reaction which may be performed by applying a method according to the invention.

In Examples 1 and 2, it is shown exemplary that the use of a solid phase (tC18) and an elevated temperature (40-75°C) is able to induce a reaction of radioactive compound (Boc₂-[¹³¹I]-SGMIB).

A **chemical reaction** of a compound is defined as the conversion of said compound to a product, wherein said conversion involves the formation and/or the breakage of one or more of the (chemical) bonds in said compound. Moreover, said compound and product may have the same number of chemical bonds and/or the same types of chemical bonds. For example, a chemical reaction of a compound may be a racemization or an isomerization of said compound. However, the interconversion of conformers is not considered a chemical reaction, insofar as it only involves the rotation around covalent bonds and not the breakage/formation of any bonds. In the context of this application the terms chemical reaction and reaction are used interchangeably. A product of a chemical reaction is defined herein as any compound which is formed during said chemical reaction.

A **chemical bond,** also called a bond in the context of this application, may be any type of chemical bond known to skilled person, including but not limited to a covalent bond, an ionic bond, a polar bond or a hydrogen bond. Preferably, a (chemical) bond refers to a covalent bond. According to this preferred definition, a method according to the invention results involves the formation and/or the breakage of covalent bonds.

A chemical reaction may comprise two or more chemical reactions as defined above. For example, the conjugation of an antibody fragment to a radiolabeled compound may involve the hydrolysis of a protecting group from said radiolabeled compound to form a deprotected compound, followed by the condensation of said deprotected compound and said antibody fragment. In the context of this invention, it is said that a chemical reaction may comprise several reaction steps. A **reaction step** of a given reaction is defined as any reaction comprised in said given reaction. Hence, a method for performing a reaction comprises a method for performing each reaction step comprised in said reaction by definition.

A **method for performing** a chemical reaction of a radioactive compound means allowing said chemical reaction to take place. This may be done by controlling one or more environmental factors, such as the temperature, and bringing the compounds required for said reaction, such as said radioactive compound, and optionally other reactants, solvents and/or catalysts, together. Herein, controlling one or more environmental factors or bringing the compounds required for a reaction together should not be interpreted as implying that said chemical reaction proceeds as a one-pot reaction wherein all reactants, solvents and catalysts are contacted at the same time. A product of a method for performing a chemical reaction is defined herein as a product of said chemical reaction.

The **heating** step comprised in a method according to the invention comprises heating said mixture to a temperature selected in the range from 30°C up to 150°C.

Preferably, the duration of said heating is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 minutes. More preferably, the duration of said heating is selected in the range from 1 minute up to 240 minutes, up to 210 minutes, up to 180 minutes, up to 150 minutes, up to 120 minutes, up to 110 minutes, up to 100 minutes, up to 90, minutes, up to 80 minutes, up to 70 minutes, up to 60 minutes, up to 50 minutes, up to 40 minutes, up to 30 minutes, up to 20 minutes, up to 19 minutes, up to 18 minutes, up to 17 minutes, up to 16 minutes, up to 15 minutes, up to 14 minutes, up to 13 minutes, up to 12 minutes, up to 11 minutes, up to 10 minutes, up to 9 minutes, up to 8 minutes, up to 7 minutes, up to 6 minutes, up to 5 minutes.

Preferably, said heating of said mixture is to a temperature selected in the range from 30°C up to 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, 145°C, or 150°C. Preferably, said heating of said mixture is to a temperature selected in the range from 30°C, 35°C, 40°C, 45°C, 50°C, 55°C, 60°C, or 65°C up to 70°C. Preferably, said heating of said mixture is to a temperature selected in the range from 30°C, 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, or 145°C up to 150°C.

Preferably, said heating of said mixture is to a temperature selected in the range from 10°C centered around 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, or 145°C. Preferably, said heating of said mixture is to a temperature selected in the range from 20°C centered around 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, or 140°C. Preferably, said heating of said mixture is to a temperature selected in the range of 30°C centered around 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, or 135°C. Preferably, said heating of said mixture is to a temperature selected in the range of 40°C centered around 50°C, 55°C, 60°C, 65°C, 70°C, 75°C, 80°C, 85°C, 90°C, 95°C, 100°C, 105°C, 110°C, 115°C, 120°C, 125°C, or 130°C. Preferably, said heating of said mixture is to a temperature selected in the range of 50°C centered around 55°C, 60°C, 65°C, 70°C, 75°C, 80°C, 85°C, 90°C, 95°C, 100°C, 105°C, 110°C, 115°C, 120°C, or125°C.

Preferably, said heating of said mixture is to a temperature selected in the range from 30°C up to 70°C and the duration of said heating is selected in the range from 1 minute up to 15 minutes, or said heating of said mixture is to a temperature selected in the range from 30°C up to 55°C and the duration of said heating is selected in the range from 1 minute up to 15 minutes, or said heating of said mixture is to a temperature selected in the range from 30°C up to 70°C and the duration of said heating is selected in the range from 1 minute up to 10 minutes, or said heating of said mixture is to a temperature selected in the range from 30°C up to 55°C and the duration of said heating is selected in the range from 1 minute up to 10 minutes, or said heating of said mixture is to a temperature selected in the range from 30°C up to 70°C and the duration of said heating is selected in the range from 1 minute up to 5 minutes, or said heating of said mixture is to a temperature selected in the range from 30°C up to 55°C and the duration of said heating is selected in the range from 1 minute up to 5 minutes.

In the context of a method according to the invention, heating said mixture to a given temperature for a given duration means that heat is provided to said solid, used in said method, and said mixture for said given duration in order to let said mixture reach said given temperature after an equilibration period, and that the temperature of said mixture is said given temperature for the remaining part of said duration. Herein, it is understood that said equilibration period is shorter than said given duration. A mixture has reached a given temperature, or the temperature of a mixture is a given temperature, if the (spatially) mean temperature of said mixture does not deviate from said given temperature by more than 1°C, preferably by not more than 0.5°C., more preferably by not more than 0.2 °C. The (spatially) mean temperature may be measured using a simple thermometer submerged in a fluid thermally equilibrated with said mixture.

Preferably, said equilibration period is from 180 seconds, or 170 seconds, or 160 seconds, or 150 seconds, or 140 seconds, or 130 seconds, or 120 seconds, or 110 seconds, or 100 seconds, or 90 seconds, or 80 seconds, or 70 seconds, or 60 seconds, or 50 seconds, or 40 seconds, or 30 seconds, or 20 seconds, or 10 seconds down to 1 second, more preferably said equilibration period is from 60 seconds down to 1 second.

A mixture with a homogeneous temperature distribution is a mixture wherein the temperature at any point in space does not deviate from the spatial mean of the temperature of said mixture by more than 1°C, preferably by not more than 0.5°C, more preferably by not more than 0.2°C as measured by an infrared temperature sensor. In all embodiments herein, a mixture with a given temperature is preferably a mixture with a homogeneous temperature distribution.

The **yield** of a product of a chemical reaction of a radioactive compound is defined as the ratio of the number of molecules of said product formed during said reaction to the total number of molecules of said radioactive compound at the start of said reaction. In the context of this definition, it is understood that said total number includes those molecules which could be considered as said radioactive compound, and could or were able to react in said chemical reaction, but did nevertheless not lead to said product during said chemical reaction. For example, the yield of a hydrolysis reaction may be less than 100% due to unreacted radioactive compound and/or due to side reactions leading to other products. Preferably, the yield is determined via quantitative HPLC, as known to the skilled person.

Whenever reference is made to the yield of a product or a product yield, said product is a product of a chemical reaction according to the invention, and is defined as above. Moreover, unless explicitly stated otherwise, it is implicitly assumed in the application that the concentration of the radioactive compound at the start of said reaction is stoichiometrically limiting. Furthermore, the yield is defined for the conditions under which a chemical reaction takes place, including parameters such as the temperature and the duration of the heating.

The yield of a chemical reaction is defined as the yield of the desired product of said chemical reaction, wherein said desired product is specified or is clear from the context. For example, in line with the definitions below, the yield of a hydrolytic deprotection preferably refers to yield of the fully deprotected product, unless explicitly stated otherwise.

Correspondingly, the yield of a product at the end of step (b) or at the end of step (c) comprised in a method according to the invention is defined as the ratio of the number of molecules of said product obtained at the end of said step (b) or (c) to the total number of molecules of said radioactive compound at the start of said reaction.

Preferably, the yield (of a desired product) of a reaction according to the invention, or of a reaction step comprised therein, is at least 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5%. Preferably, when reference is made to the yield of a reaction or a reaction step, or of a product thereof, without specifying the conditions wherein said reaction or reaction step takes place, said yield refers to the yield after 5 or 10 minutes of heating. More preferably, when reference is made to the yield of a reaction or a reaction step, or of a product thereof, without specifying the conditions wherein said reaction or reaction step takes place, said yield refers to the yield after 5 minutes of heating at 30°C, 35°C, 40°C, 45°C, 50°C, or 55°C.

A product mixture is defined as a composition which has been isolated at the end of a reaction of a radioactive compound, with the aim of retrieving a desired product of said reaction, wherein said composition comprises said desired product, wherein said isolation may involve the use of one or more purification methods, as known to the skilled person, such as affinity purification, filtration, centrifugation, evaporation, liquid-liquid extraction, crystallization, recrystallization, adsorption, chromatography, distillation, fractionation, electrolysis, or sublimation.

The **purity** of a product of a chemical reaction of a radioactive compound is defined as the concentration of said product in a product mixture. Preferably, the purity is the ratio of the mass of said product comprised in said product mixture to the total mass of said product mixture. The purity is determined using similar methods as the yield, i.e. by determining the concentration of the desired product by quantitative HPLC or TLC, as known to the skilled person. Whenever reference is made to the purity of a product or a product purity, said product is a product of a chemical reaction according to the invention, and is defined as above.

Preferably, the purity of a desired product of a reaction according to the invention, or of a reaction step comprised therein, is at least 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5%.

Chemical reactions, and particularly chemical reactions of radioactive compounds, are often characterized by a high **activation barrier.** A chemical reaction with a high activation barrier is defined as a chemical reaction which, in order to obtain a yield of a desired product of at least 50%, has to be performed in the absence of a catalyst or a facilitator, preferably in the absence of a solid phase which is able to act as a facilitator during said chemical reaction, for 30 minutes at 50°C, or for 30 minutes at 60°C, or for 30 minutes at 70°C, or for 30 minutes at 80°C, or for 30 minutes at 90°C, or for 30 minutes at 100°C, or for 30 minutes at 110°C, or for 30 minutes at 120°C, or for 30 minutes at 130°C, or for 30 minutes at 140°C, or for 30 minutes at 150°C, or for 60 minutes at 50°C, or for 60 minutes at 60°C, or for 60 minutes at 70°C, or for 60 minutes at 80°C, or for 60 minutes at 90°C, or for 60 minutes at 100°C, or for 60 minutes at 110°C, or for 60 minutes at 120°C, or for 60 minutes at 130°C, or for 60 minutes at 140°C, or for 60 minutes at 150°C, or for 90 minutes at 50°C, or for 90 minutes at 60°C, or for 90 minutes at 70°C, or for 90 minutes at 80°C, or for 90 minutes at 90°C, or for 90 minutes at 100°C, or for 90 minutes at 110°C, or for 90 minutes at 120°C, or for 90 minutes at 130°C, or for 90 minutes at 140°C, or for 90 minutes at 150°C, or for 120 minutes at 50°C, or for 120 minutes at 60°C, or for 120 minutes at 70°C, or for 120 minutes at 80°C, or for 120 minutes at 90°C, or for 120 minutes at 100°C, or for 120 minutes at 110°C, or for 120 minutes at 120°C, or for 120 minutes at 130°C, or for 120 minutes at 140°C, or for 120 minutes at 150°C, or for 150 minutes at 50°C, or for 150 minutes at 60°C, or for 150 minutes at 70°C, or for 150 minutes at 80°C, or for 150 minutes at 90°C, or for 150 minutes at 100°C, or for 150 minutes at 110°C, or for 150 minutes at 120°C, or for 150 minutes at 130°C, or for 150 minutes at 140°C, or for 150 minutes at 150°C, or for 180 minutes at 50°C, or for 180 minutes at 60°C, or for 180 minutes at 70°C, or for 180 minutes at 80°C, or for 180 minutes at 90°C, or for 180 minutes at 100°C, or for 180 minutes at 110°C, or for 180 minutes at 120°C, or for 180 minutes at 130°C, or for 180 minutes at 140°C, or for 180 minutes at 150°C, or for 210 minutes at 50°C, or for 210 minutes at 60°C, or for 210 minutes at 70°C, or for 210 minutes at 80°C, or for 210 minutes at 90°C, or for 210 minutes at 100°C, or for 210 minutes at 110°C, or for 210 minutes at 120°C, or for 210 minutes at 130°C, or for 210 minutes at 140°C, or for 210 minutes at 150°C, or for 240 minutes at 50°C, or for 240 minutes at 60°C, or for 240 minutes at 70°C, or for 240 minutes at 80°C, or for 240 minutes at 90°C, or for 240 minutes at 100°C, or for 240 minutes at 110°C, or for 240 minutes at 120°C, or for 240 minutes at 130°C, or for 240 minutes at 140°C, or for 240 minutes at 150°C. In a preferred embodiment is provided a method according to the invention, wherein said chemical reaction has a high activation barrier.

In the context of this application, a **catalyst** is a compound which increases the reaction rate of a reaction according to the invention, wherein said catalyst forms a coordinative or equivalent bond, preferably a covalent bond, with one or more of the reactants during said reaction according to the invention. In the context of this application, a facilitator is a compound which increases the reaction rate of a reaction according to the invention, wherein said facilitator does not form a covalent bond with a reactant during said reaction according to the invention. Preferably, a catalyst or a facilitator is defined as a compound which lowers the temperature which is needed to perform a reaction according to the invention compared to the same reaction in the absence of said catalyst or said facilitator in the same time to obtain the same yield of a product, wherein the same reaction means using the same reactants, in essentially the same amounts. Said temperature is preferably lowered by at least 5°C, 10°C, 15°C, 20°C, 25°C, 30°C, 35°C, 40°C, 45°C, 50°C, 55°C, 60°C, 65°C, 70°C, 75°C, 80°C, 85°C, 90°C, 95°C or 100°C. In this definition, said chemical reaction according to the invention is preferably a chemical reaction with a high activation barrier. A skilled person understands that a catalyst or a facilitator, as defined above, may be used to lower the temperature and/or shorten the reaction time needed to perform a reaction to obtain the same yield of a product using the same reactants.

Furthermore, a skilled person understands that a catalyst or a facilitator may act via different mechanisms. Preferably, said solid phase used in a method according to the invention is a facilitator for said chemical reaction of said radioactive compound, wherein said facilitator increases the reaction rate of said reaction by increasing the local concentration of said radioactive compound and/or of other reactants, products or other compounds involved in said reaction.

In a method according to the invention, said radioactive compound is comprised in a **mixture.** Said mixture is a solution or a colloid comprising said radioactive compound. Preferably, said mixture is an aqueous solution or an aqueous colloid. More preferably, said mixture is an aqueous solution having a pH lower than 14, 13.5, 13, 12.5, 12, 11.5, 11, 10.5, 10, 9.5, 9, 8.5, 8, 7.5, 7, 6.5, 6, 5.5, 5, 4.5, or 4. Preferably, said mixture comprises a facilitator for performing said chemical reaction, more preferably wherein said facilitator is a solid phase as defined herein.

A **solid phase** used in a method according to the invention may be any compound in the solid state which is suitable for contacting with said mixture comprising a radioactive compound during a reaction as defined above. Suitable in this context means that said solid phase should allow for said reaction to take place under the desired or specified reaction conditions. For example, if a reaction of a radioactive compound should take place in a given solvent at a given temperature, said solid phase should not degrade in said solvent at said temperature, and preferably said solid phase should act as a facilitator thereby allowing said reaction to take place at said temperature.

In a preferred embodiment is provided a method according to the invention In a preferred embodiment is provided a method according to the invention wherein said solid phase is a facilitator for said chemical reaction of said radioactive compound. Preferably, said chemical reaction has a high activation barrier. In general, and specifically in the context of this preferred embodiment, it is understood that a reaction may have multiple catalysts and/or facilitators. Hence, said solid phase being a facilitator for said chemical reaction neither excludes nor implies the presence of other catalysts and/or facilitators during said reaction or in said mixture.

An **alkaline solution** is defined as a solution of a strong base in a solvent. Preferably, said strong base is a strong organic base, such as triethyl amine, or a strong inorganic base, more preferably a hydroxide salt of an alkali metal such as lithium (Li), sodium (Na), potassium (K), rubidium (Rb), caesium (Cs), or francium (Fr), or a hydroxide salt of an alkaline earth metal such as beryllium (Be), magnesium (Mg), calcium (Ca), strontium (Sr), barium (Ba) or radium (Ra). Preferably, said solvent is water or an organic base, such as tetrabutyl ammonium carbonate (TBAC).

More preferably, an alkaline solution is an aqueous solution having a pH of at least 8, 8.2, 8.4, 8.6, 8.8, 9, 9.2, 9.4, 9.6, 9.8, 10, 10.2, 10.4, 10.6, 10.8, 11, 11.2, 11.4, 11.6, 11.8, 12, 12.2, 12.4, 12.6, 12.8, 13, 13.2, 13.4, 13.6, 13.8 or 14. According to this preferred definition, steps (a) and (b) of a method according to the invention do not involve contacting said solid phase with an aqueous solution having a pH higher than 14, 13.8, 13.6, 13.4, 13.2, 13, 12.8, 12.6, 12.4, 12.2, 12, 11.8, 11.6, 11.4, 11.2, 11, 10.8, 10.6, 10.4, 10.2, 10, 9.8, 9.6, 9.4, 9.2, 9, 8.8, 8.6, 8.4, 8.2, or 8.

In the context of this application, the feature "wherein steps (a) and (b) do not involve contacting said solid phase with an alkaline solution" as stipulated with regards to a method according the invention may be replaced by "wherein during steps (a) and (b) any solution contacting said solid phase is a neutral or an acidic solution". Hence, a method according to the invention may thus be formulated as: a method for performing a chemical reaction of a radioactive compound comprised in a mixture, wherein said method comprises the following steps: (a) contacting said mixture with a solid phase, followed by (b) heating said mixture to a temperature selected in the range from 30°C up to 150°C, wherein during steps (a) and (b) any solution contacting said solid phase is a neutral or an acidic solution, wherein said chemical reaction does not result in the formation of a new bond on a radionuclide comprised in said radioactive compound, wherein said radioactive compound does not comprise fluorine-18.

In a preferred embodiment is provided a method according to the invention, wherein during step (b) said solid phase is in contact with said mixture comprising said radioactive compound. Hence, according to this embodiment, said mixture comprising said radioactive compound is not an alkaline solution. In other words, according to this embodiment, said mixture comprising said radioactive compound is a neutral or an acidic solution.

In a preferred embodiment is provided a method according to the invention, wherein any heating (step) which involves contacting a solution with said solid phase does not involve contacting said solid phase with an alkaline solution. In other words, according to this embodiment, any solution which is contacted with said solid phase during a heating (step) is a neutral or an acidic solution.

In a preferred embodiment is provided a method according to the invention wherein said chemical reaction does not comprise a reaction step wherein an alkaline solution is used and/or does not comprise contacting said solid phase with an alkaline solution. Preferably, an alkaline solution in the context of this embodiment is an aqueous solution having a pH of at least 8, 8.2, 8.4, 8.6, 8.8, 9, 9.2, 9.4, 9.6, 9.8, 10, 10.2, 10.4, 10.6, 10.8, 11, 11.2, 11.4, 11.6, 11.8, 12, 12.2, 12.4, 12.6, 12.8, 13, 13.2, 13.4, 13.6, 13.8 or 14.

Whenever it is said that a chemical reaction, a reaction step or a part thereof does not involve or does not comprise the use of a given compound, composition, or a mixture thereof, it is meant that said compound, composition, or said mixture thereof, are not present in, or are present in a concentration of less than 0.1 wt%, 0.01 wt%, 0.001 wt% or 0.0001wt% in, or could not be detected using quantitative HPLC in the reaction medium during said reaction, reaction step or part thereof and are not, or are not in direct contact with, the reactants and/or the products of said reaction, reaction step or part thereof. For example, a reaction of a radioactive compound comprised in a mixture which does not involve an alkaline solution cannot occur in the presence of a dissolved strong base. Nevertheless, an alkaline solution may still be used insofar that it does not come into direct contact with said mixture.

A **radioactive compound** is a compound comprising a radionuclide. A **radionuclide** is a thermodynamically unstable nucleus, which spontaneously converts to a stable nucleus via internal conversion of by the creation of a radiation particle. The conversion of said radionuclide to a stable nucleus is called radioactive decay, resulting in the emission of gamma ray(s) (γ) and/or subatomic particles such as alpha (α) or beta (β) particles. A beta (β) particle may be an electron (β⁻) or a positron (β⁺). These emissions constitute ionizing radiation. In this application, the terms radionuclide, radioactive nuclide, radioisotope and radioactive isotope may be used interchangeably. Moreover, a radionuclide also refers to an atom comprising said radionuclide. For example, a bond on or with a radionuclide, or a salt of a radionuclide, are defined in this sense. Preferably, a radioactive compound comprises not more than one radionuclide.

The **loss of a radionuclide** comprised in a radioactive compound, also called radiolysis or radiolytic degradation of said radioactive compound, is defined as the breakage of the bonds between said radionuclide and the other atoms comprised in said radioactive compound or the breakage of the bonds between the other atoms of said radioactive compound caused by the linear energy transfer of the radionuclide, whereafter said radionuclide is no longer a part of said compound or bound to only a fragment of said radioactive compound. In the preferred case wherein a radioactive compound comprises only one radionuclide, and not more than one, the loss of a radionuclide leads to a non-radioactive compound and the released radionuclide or a fraction of the radioactive compound comprising the radionuclide. In light of this definition, the skilled person understands that said fraction of said radioactive compound is considered to be an unwanted or side product. For example, in case of a deprotection reaction of a (protected) radioactive compound, the deprotected product is not considered is to be a fraction of said radioactive compound.

The **degree of radiolysis** of or during a chemical reaction of a radioactive compound is defined as the ratio between the number of molecules of said radioactive compound which undergo loss of a radionuclide during said chemical reaction to the total number of molecules of said radioactive compound at the start of said chemical reaction.

Correspondingly, the degree of radiolysis may be defined during any reaction step comprised in a reaction according to the invention, or during any step ortime interval comprised in a reaction according to the invention or in a method according to the invention. As one example, the degree of radiolysis during the heating (b) comprised in a method according to the invention is defined as the ratio between the number of molecules of said radioactive compound which undergo loss of a radionuclide during said heating to the total number of molecules of said radioactive compound at the start of said heating.

Preferably, during a reaction according to the invention said radioactive compound is converted into a radioactive product, i.e. a product comprising a radionuclide. More preferably, said radioactive compound and said radioactive product comprise the same radionuclide(s). In other words, the loss of a radionuclide of said radioactive compound may be considered undesired side reaction in a reaction according to the invention.

In a preferred embodiment is provided a method according to the invention wherein the degree of radiolysis during said chemical reaction is lower than 5%, 4.5%, 4%, 3.5%, 3%, 2.5%, 2%, 1.5%, 1%, 0,9%, 0.8%, 0.7%, 0.6%, 0.5%, 0.4%, 0.3%, 0.2%, or 0.1%.

In a preferred embodiment is provided a method according to the invention wherein said chemical reaction does not involve the loss of a radionuclide comprised in said radioactive compound.

In a preferred embodiment is provided a method according to the invention wherein the degree of radiolysis during said heating (b) is lower than 5%, 4.5%, 4%, 3.5%, 3%, 2.5%, 2%, 1.5%, 1%, 0,9%, 0.8%, 0.7%, 0.6%, 0.5%, 0.4%, 0.3%, 0.2%, or 0.1%. Preferably, said degree of radiolysis refers to the yield after 5 or 10 minutes of heating, more preferably to the degree of radiolysis after 5 minutes of heating at 30°C, 35°C, 40°C, 45°C, 50°C, or 55°C.

In a preferred embodiment is provided a method according to the invention wherein said heating (b) does not involve the radioactive decay of said compound and/or the loss of a radionuclide comprised in said radioactive compound.

Moreover, a radioactive compound is prone to **radioactive decay,** which is defined as the radioactive decay of at least one of the radionuclides comprised in said radioactive compound, preferably of all radionuclides comprised in said radioactive compound. A radioactive compound is called an α-emitter, a β-emitter, or a γ-emitter or an Auger-emitter if said compound comprises a radionuclide which may undergo radioactive decay by emitting α particles, β particles, or γ rays or Auger-electrons, respectively. Herein, it is understood that a radioactive compound may emit more than one type of particles or radiation.

Since a radioactive compound, as used in a method according to the invention, is prone to both radiolysis and radioactive decay, the contact-time of all reactants should be reduced to a minimum.

A **post-labeling** (chemical) reaction of a radioactive compound is defined as a chemical reaction of said radioactive compound which does not result in the formation of a new bond on a radionuclide comprised in said radioactive compound. For example, the transfer of a radionuclide between two compounds via a nucleophilic substitution would not be considered a post-labeling reaction, whereas a nucleophilic substitution of another moiety comprised in a radioactive compound may be considered a post-labeling reaction. Without being bound to this theory, the preparation of a radioactive compound of interest typically involves a labeling reaction, wherein a radionuclide is attached to a non-radioactive compound to form a radioactive intermediate, followed by one or more post-labeling reactions of said radioactive intermediate, wherein said post-labeling reactions do not alter the nature of the attachment of said radionuclide to said intermediate, as understood by the skilled person.

A reaction according to the invention is a post-labeling reaction. In the context of this application the feature "wherein said chemical reaction does not result in the formation of a new bond on a radionuclide comprised in said radioactive compound" as stipulated with regards to a method according to the invention may be replaced by "wherein said chemical reaction is a post-labeling reaction". In other words, a method according to the invention may also be also be formulated as: a method for performing a chemical reaction of a radioactive compound comprised in a mixture, wherein said method comprises the following steps: (a) contacting said mixture with a solid phase, followed by (b) heating said mixture to a temperature selected in the range from 30°C up to 150°C, wherein steps (a) and (b) do not involve contacting said solid phase with an alkaline solution, wherein said chemical reaction is a post-labeling reaction, wherein said radioactive compound does not comprise fluorine-18.

In view of the above, a method according to the invention may also be also be formulated as: a method for performing a chemical reaction of a radioactive compound comprised in a mixture, wherein said method comprises the following steps: (a) contacting said mixture with a solid phase, followed by (b) heating said mixture to a temperature selected in the range from 30°C up to 150°C, wherein during steps (a) and (b) any solution contacting said solid phase is a neutral or an acidic solution, wherein said chemical reaction is a post-labeling reaction, wherein said radioactive compound does not comprise fluorine-18.

It is well known to the skilled person that a radioactive compound may comprise a stereogenic center, i.e. an asymmetrically substituted atom wherein said asymmetric substitution gives rise to **chiral properties** such as the rotation of plane-polarized light or the stereospecific or -selective catalysis by enzymes. Preferably, a stereogenic center is an asymmetrically substituted, tetravalent carbon atom. Each stereogenic center comprised in said radioactive compound may exist in one of two (stereo)configurations, wherein said configurations do not differ in the type and the amount of bonds between the atoms comprised in said radioactive compound. The epimerization of a stereogenic center comprised in a radioactive compound is defined as the conversion of one configuration of said stereogenic center into the other. The enantiomerization of a radioactive compound is defined as the epimerization of all stereogenic centers in said radioactive compound. The diastereoisomerization of a radioactive compound is defined as the epimerization of at least one but not all stereogenic centers in said radioactive compound.

In a preferred embodiment is provided a method according to the invention, wherein said chemical reaction does not comprise an epimerization, or a diastereoisomerization, or an enantiomerization of said radioactive compound.

In a more preferred embodiment is provided a method according to the invention, wherein said (a) contacting with a solid phase and/or (b) said heating do not comprise an epimerization, or a diastereoisomerization, or an enantiomerization of said radioactive compound.

In another more preferred embodiment is provided a method according to the invention, wherein any epimerization reaction comprised in, i.e. occurring during, said (a) contacting with a solid phase and/or (b) said heating, has a yield lower than 50%, 45%, 40%, 35%, 30%, 25%, 20%, 19%, 18%, 17%, 16%, 15%, 14%, 13%, 12%, 11%, 10%, 9%, 8%, 7%, 6%, 5%,4%, 3%, 2%, 1%, 0.9%, 0.8%, 0.7%, 0.6%, 0.5%, 0.4%, 0.3%, 0.2%, or 0.1%.

### Reaction types

In a preferred embodiment is provided a method according to the invention wherein said chemical reaction comprises a **solvolysis** of said radioactive compound, preferably wherein said solvolysis occurs during said heating. A solvolysis of said radioactive compound is a reaction between said radioactive compound and a solvent, wherein said reaction comprises the breakage of a bond in said radioactive compound, preferably wherein said reaction comprises the formation a bond between the atoms comprised in said solvent and the atoms comprised in said radioactive compound. Examples of a solvolysis include transesterifications, the displacement or substitution of a leaving group by a solvent, dehydrations and hydrolyses.

A solvolysis preferably means a reaction between a nucleophilic solvent and said radioactive compound, wherein a bond is formed between an atom comprised in said radioactive compound and an atom comprised in said nucleophilic solvent, wherein said reaction can be described as a nucleophilic substitution reaction or elimination reaction of said radioactive compound, preferably wherein said nucleophilic solvent is selected from the group consisting of water and alcohols, most preferably said nucleophilic solvent is water. Preferably, said solvolysis does not comprise the use of an alkaline solution.

In a more preferred embodiment is provided a method according to the invention wherein said chemical reaction comprises a solvolysis of said radioactive compound, wherein said solvolysis occurs during said heating, wherein said solvolysis has a high activation barrier, preferably wherein said solid phase is a facilitator for said solvolysis.

In the preferred embodiments above, the degree of radiolysis during said solvolysis is preferably lower than 5%, 4.5%, 4%, 3.5%, 3%, 2.5%, 2%, 1.5%, 1%, 0,9%, 0.8%, 0.7%, 0.6%, 0.5%, 0.4%, 0.3%, 0.2%, or 0.1%. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

As well known to the skilled person, a solvolysis yields to a solvolysis product. In the preferred embodiments above, the yield of said solvolysis product is at least 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 92.5%, 95%, or 97.5%. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In a most preferred embodiment is provided a method according to the invention wherein said chemical reaction comprises a solvolysis of said radioactive compound, wherein said solvolysis occurs during said heating, wherein the degree of radiolysis during said solvolysis is lower than 5% and wherein the yield of said solvolysis product is at least 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 92.5%, 95%, or 97.5%, preferably wherein the degree of radiolysis during said solvolysis is lower than 3% and wherein the yield of said solvolysis product is at least 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 92.5%, 95%, or 97.5%. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In a more preferred embodiment is provided a method according to the invention wherein said chemical reaction comprises a **hydrolysis** of said radioactive compound, preferably wherein said hydrolysis occurs during said heating. Preferably, said hydrolysis occurs in an aqueous solution at a pH lower than 7, 6.8, 6.6, 6.4, 6.2, 6, 5.8, 5.6, 5.4, 5.2 or 5. More preferably, said hydrolysis occurs in an aqueous solution at a pH from 14, 13.8, 13.6, 13.4, 13.2, 13, 12.8, 12.6, 12.4, 12.2, 12, 11.8, 11.6, 11.4, 11.2, 11, 10.8, 10.6, 10.4, 10.2, 10, 9.8, 9.6, 9.4, 9.2, 9, 8.8, 8.6, 8.4, 8.2, 8, 7.8, 7.6, 7.4, 7.2, 7, 6.8, 6.6, 6.4, 6.2, 6, 5.8, 5.6, 5.4, 5.2 or 5 down to 1. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In an even more preferred embodiment is provided a method according to the invention wherein said chemical reaction comprises a hydrolysis of said radioactive compound, preferably wherein said hydrolysis occurs during said heating, wherein said hydrolysis is an acid hydrolysis, preferably wherein said acid hydrolysis comprises the use of an acid selected from the group consisting of phosphoric acid, hydrochloric acid, sulfuric acid, trifluoroacetic acid and aqueous mixtures thereof, more preferably wherein said acid is selected from the group consisting of phosphoric acid, hydrochloric acid, sulfuric acid, and aqueous mixtures thereof, preferably wherein said acid is 50 wt%, 60 wt%, 70 wt%, 80 wt%, 90 wt% phosphoric acid, more preferably wherein said acid is 80 wt% phosphoric acid. Preferably, said hydrolysis occurs in an aqueous solution at a pH lower than 7, 6.8, 6.6, 6.4, 6.2, 6, 5.8, 5.6, 5.4, 5.2 or 5. More preferably, said hydrolysis occurs in an aqueous solution at a pH from 14, 13.8, 13.6, 13.4, 13.2, 13, 12.8, 12.6, 12.4, 12.2, 12, 11.8, 11.6, 11.4, 11.2, 11, 10.8, 10.6, 10.4, 10.2, 10, 9.8, 9.6, 9.4, 9.2, 9, 8.8, 8.6, 8.4, 8.2, 8, 7.8, 7.6, 7.4, 7.2, 7, 6.8, 6.6, 6.4, 6.2, 6, 5.8, 5.6, 5.4, 5.2 or 5 down to 1. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In a more preferred embodiment is provided a method according to the invention wherein said chemical reaction comprises a hydrolysis of said radioactive compound, wherein said hydrolysis occurs during said heating, wherein said hydrolysis has a high activation barrier, preferably wherein said solid phase is a facilitator for said hydrolysis. Preferably, the rate of said hydrolysis is further enhanced by the use of an acid selected from the group consisting of phosphoric acid, hydrochloric acid, sulfuric acid, trifluoroacetic acid and aqueous mixtures thereof, more preferably by an acid selected from the group consisting of phosphoric acid, hydrochloric acid, sulfuric acid, and aqueous mixtures thereof, preferably wherein said acid is 50 wt%, 60 wt%, 70 wt%, 80 wt%, 90 wt% phosphoric acid, more preferably wherein said acid is 80 wt% phosphoric acid. Preferably, said hydrolysis occurs in an aqueous solution at a pH lower than 14, 13.8, 13.6, 13.4, 13.2, 13, 12.8, 12.6, 12.4, 12.2, 12, 11.8, 11.6, 11.4, 11.2, 11, 10.8, 10.6, 10.4, 10.2, 10, 9.8, 9.6, 9.4, 9.2, 9, 8.8, 8.6, 8.4, 8.2, 8, 7.8, 7.6, 7.4, 7.2, 7, 6.8, 6.6, 6.4, 6.2, 6, 5.8, 5.6, 5.4, 5.2 or 5. More preferably, said hydrolysis occurs in an aqueous solution at a pH from 14, 13.8, 13.6, 13.4, 13.2, 13, 12.8, 12.6, 12.4, 12.2, 12, 11.8, 11.6, 11.4, 11.2, 11, 10.8, 10.6, 10.4, 10.2, 10, 9.8, 9.6, 9.4, 9.2, 9, 8.8, 8.6, 8.4, 8.2, 8, 7.8, 7.6, 7.4, 7.2, 7, 6.8, 6.6, 6.4, 6.2, 6, 5.8, 5.6, 5.4, 5.2 or 5 down to 1. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In the preferred embodiments above, the degree of radiolysis during said hydrolysis is preferably lower than 5%, 4.5%, 4%, 3.5%, 3%, 2.5%, 2%, 1.5%, 1%, 0,9%, 0.8%, 0.7%, 0.6%, 0.5%, 0.4%, 0.3%, 0.2%, or 0.1%. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In the preferred embodiments above, the degree of radiolysis during said hydrolysis is preferably lower than 5%, wherein said hydrolysis occurs during heating, wherein the duration of said heating is in the range from 1 minute up to 10 minutes, more preferably in the range from 1 minutes up to 5 minutes, and said heating of said mixture is to a temperature selected in the range from 30°C up to 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, 145°C, or 150°C. Preferably, the duration of said heating is in the range from 1 minute up to 10 minutes, more preferably in the range from 1 minute up to 5 minutes, and said heating of said mixture is to a temperature selected in the range from 30°C, 35°C, 40°C, 45°C, 50°C, 55°C, 60°C, 65°C, 70°C, or 75°C up to 70°C.

As well known to the skilled person, a hydrolysis yields to a hydrolysis product. Such a product may be for example a hydrolytically deprotected radioactive compound, or a carboxylic acid, or salt or ion thereof, due to the hydrolysis of an amide or ester group. In the preferred embodiments above, the yield of said hydrolysis product is at least 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 92.5%, 95%, or 97.5%. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In an even more preferred embodiment is provided a method according to the invention wherein said chemical reaction comprises a hydrolysis of said radioactive compound, wherein said hydrolysis occurs during said heating, wherein the degree of radiolysis during said hydrolysis is lower than 5% and wherein the yield of said hydrolysis product is at least 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 92.5%, 95%, or 97.5%, preferably wherein the degree of radiolysis during said hydrolysis is lower than 3% and wherein the yield of said hydrolysis product is at least 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 92.5%, 95%, or 97.5%. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In a most preferred embodiment is provided a method according to the invention wherein said chemical reaction comprises a hydrolysis of said radioactive compound, wherein said hydrolysis occurs during said heating, wherein the degree of radiolysis during said hydrolysis is lower than 5% and wherein the yield of said hydrolysis product is at least 30%, preferably wherein the degree of radiolysis during said hydrolysis is lower than 3% and wherein the yield of said hydrolysis product is at least 35%, wherein the duration of said heating is in the range from 1 minute up to 10 minutes, more preferably in the range from 1 minute up to 5 minutes, and said heating of said mixture is to a temperature selected in the range from 30°C up to 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, 145°C, or 150°C. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes, or said heating of said mixture is to a temperature selected in the range from 30°C up to 55°C and the duration of said heating is selected in the range from 1 minute up to 5 minutes. Most preferably, said heating of said mixture is to a temperature selected in the range from 30°C up to 55°C and the duration of said heating is selected in the range from 1 minute up to 10 minutes.

In another preferred embodiment is provided a method according to the invention wherein said chemical reaction comprises a hydrolysis of said radioactive compound, wherein said hydrolysis occurs during said heating, wherein the degree of radiolysis during said hydrolysis is lower than 5% and wherein the yield of said hydrolysis product is at least 30%, preferably wherein the degree of radiolysis during said hydrolysis is lower than 3% and wherein the yield of said hydrolysis product is at least 35%, wherein said hydrolysis occurs in an aqueous solution at a pH lower than 14, 13.8, 13.6, 13.4, 13.2, 13, 12.8, 12.6, 12.4, 12.2, 12, 11.8, 11.6, 11.4, 11.2, 11, 10.8, 10.6, 10.4, 10.2, 10, 9.8, 9.6, 9.4, 9.2, 9, 8.8, 8.6, 8.4, 8.2, 8, 7.8, 7.6, 7.4, 7.2, 7, 6.8, 6.6, 6.4, 6.2, 6, 5.8, 5.6, 5.4, 5.2 or 5. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In another more preferred embodiment is provided a method according to the invention wherein said chemical reaction comprises a hydrolysis of said radioactive compound, wherein said hydrolysis occurs during said heating, wherein the degree of radiolysis during said hydrolysis is lower than 5% and wherein the yield of said hydrolysis product is at least 30%, preferably wherein the degree of radiolysis during said hydrolysis is lower than 3% and wherein the yield of said hydrolysis product is at least 35%, wherein said hydrolysis occurs in an aqueous solution at a pH lower than 7, wherein the duration of said heating is in the range from 1 minute up to 10 minutes, more preferably in the range from 1 minute up to 5 minutes, and said heating of said mixture is to a temperature selected in the range from 30°C up to 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, 145°C, or 150°C. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes, or said heating of said mixture is to a temperature selected in the range from 30°C up to 55°C and the duration of said heating is selected in the range from 1 minute up to 5 minutes. Most preferably, said heating of said mixture is to a temperature selected in the range from 30°C up to 55°C and the duration of said heating is selected in the range from 1 minute up to 10 minutes.

In a most preferred embodiment is provided a method according to the invention wherein said chemical reaction comprises a hydrolysis of said radioactive compound, wherein said hydrolysis occurs during said heating, wherein the yield of said hydrolysis product is at least 30%, preferably wherein the yield of said hydrolysis product is at least 35%, wherein said hydrolysis occurs in an aqueous solution at a pH lower than 7, wherein the duration of said heating is in the range from 1 minute up to 10 minutes, more preferably in the range from 1 minute up to 5 minutes, and said heating of said mixture is to a temperature selected in the range from 30°C up to 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, 145°C, or 150°C. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes, or said heating of said mixture is to a temperature selected in the range from 30°C up to 55°C and the duration of said heating is selected in the range from 1 minute up to 5 minutes. Most preferably, said heating of said mixture is to a temperature selected in the range from 30°C up to 55°C and the duration of said heating is selected in the range from 1 minute up to 10 minutes.

Said radioactive compound used in a method according to the invention may comprise one or more **protecting groups.** As well known to the skilled person, a protecting group is a functional group which is covalently bonded to said radioactive compound and serves the purpose of directing the chemoselectivity of said chemical reaction, i.e. of obtaining a desired final product with a given yield, wherein said functional group is not covalently bonded to said desired final product.

Preferably, a protecting group is selected from the group consisting of tert-butylcarbamate (t-Boc), tert-buylester (OtBu), Benzylester (BzO), benzylidene, tetrahydropyranyl ether (THP), acetal, trityl (Trt) and methoxymethyl ether (MOM). Most preferably, a protecting group is tert-butylcarbamate (t-Boc).

The removal of a protecting group (from a radioactive compound) is defined as a chemical reaction of said radioactive compound wherein the covalent bonds between said protecting group and the other atoms comprised in said radioactive compound is broken. The product of such a removal is called a deprotected radioactive compound. Removal of a protecting group may also be called deprotection in the context of this application. Solvolyses and hydrolyses as defined above preferably comprise the removal of a protecting group from a radioactive compound.

In a preferred embodiment is provided a method according to the invention, wherein said chemical reaction comprises a solvolysis of said radioactive compound, wherein said solvolysis occurs during said heating, wherein said solvolysis comprises a removal of a protecting group from said radioactive compound, preferably wherein said protecting group is selected from the group consisting of tert-butylcarbamate (t-Boc), tert-buylester (OtBu), Benzylester (BzO), benzylidene, tetrahydropyranyl ether (THP), acetal, trityl (Trt) and methoxymethyl ether (MOM), most preferably wherein said protecting group is tert-butylcarbamate (t-Boc).

In a preferred embodiment is provided a method according to the invention, wherein said chemical reaction comprises a hydrolysis of said radioactive compound, wherein said hydrolysis occurs during said heating, wherein said hydrolysis comprises a removal of a protecting group from said radioactive compound, preferably wherein said protecting group is selected from the group consisting of tert-butylcarbamate (t-Boc), tert-buylester (OtBu), Benzylester (BzO), benzylidene, tetrahydropyranyl ether (THP), acetal, trityl (Trt) and methoxymethyl ether (MOM), most preferably wherein said protecting group is tert-butylcarbamate (t-Boc).

As explained above, a radioactive compound may comprise more than one protecting group. In an embodiment is provided a method according to the invention wherein said radioactive compound comprises exactly one, or two, or three, or four protecting groups, and not more than said number. In another embodiment is provided a method according to the invention wherein said radioactive compound comprises more than one protecting groups. A radioactive compound used in the latter preferred method is called a radioactive compound with multiple protecting groups.

The complete removal of protecting groups from, also called the complete or full deprotection of, a radioactive compound is defined as the removal of all protecting groups comprised in said radioactive compound having multiple protecting groups. The product of said complete removal is called a fully deprotected radioactive compound. Correspondingly, the incomplete removal of protecting groups from, also called the incomplete or partial deprotection of, a radioactive compound is defined as the removal of at least one but not all protecting groups comprised in said radioactive compound having multiple protecting groups. The product of said incomplete removal is called a partially deprotected radioactive compound.

In a preferred embodiment is provided a method according to the invention wherein said chemical reaction comprises a hydrolysis of said radioactive compound, wherein said hydrolysis occurs during said heating, wherein said hydrolysis comprises a removal of a protecting group from said radioactive compound, wherein said radioactive compound has multiple protecting groups independently selected from the group consisting of tert-butylcarbamate (t-Boc), tert-buylester (OtBu), Benzylester (BzO), benzylidene, tetrahydropyranyl ether (THP), acetal, trityl (Trt) and methoxymethyl ether (MOM), preferably wherein each protecting group is tert-butylcarbamate (t-Boc). Preferably, the yield of the corresponding fully deprotected radioactive compound is at least 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 92.5%, 95% or 97.5%. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In a more preferred embodiment is provided a method according to the invention wherein said chemical reaction comprises a hydrolysis of said radioactive compound, wherein said hydrolysis occurs during said heating, wherein said hydrolysis comprises a removal of a protecting group from said radioactive compound,
- wherein the degree of radiolysis during said hydrolysis is lower than 5%, preferably lower than 3%; and/or
- wherein the yield of the corresponding fully deprotected radioactive compound is at least 30%, preferably at least 35%; and/or
- wherein said hydrolysis occurs in an aqueous solution at a pH lower than 7, preferably lower than 6; and/or
- wherein said mixture comprises phosphoric acid; and/or
- wherein said radioactive compound has multiple protecting groups independently selected from the group consisting of tert-butylcarbamate (t-Boc), tert-buylester (OtBu), Benzylester (BzO), benzylidene, tetrahydropyranyl ether (THP), acetal, trityl (Trt) and methoxymethyl ether (MOM), preferably wherein each protecting group is tert-butylcarbamate (t-Boc); and/or
- wherein the duration of said heating is in the range from 1 minute up to 10 minutes, more preferably in the range from 1 minute up to 5 minutes; and/or
- wherein said heating of said mixture is to a temperature selected in the range from 30°C up to 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, 145°C, or 150°C.

A side reaction of a removal of a protecting group from a radioactive compound is defined as a chemical reaction of said radioactive compound which leads to a side product, wherein said side product is not said radioactive compound, the partially deprotected radioactive compound corresponding to said removal, or the fully deprotected radioactive compound corresponding to said removal. For example, an epimerization may be considered a side reaction a removal of a protecting group. Herein, it is understood that "corresponding to said removal" implies that a side reaction may be the removal of another type of protecting group. For example, the removal of a tetrahydropyranyl ether (THP) protecting group may be considered as a side reaction of the removal of a tert-butylcarbamate (t-Boc) protecting group. In line with the definition above, a side product of a removal of a protecting group from a radioactive compound is defined as a compound formed during a side reaction of said removal, wherein said side product is not said radioactive compound, the partially deprotected radioactive compound corresponding to said removal, or the fully deprotected radioactive compound corresponding to said removal.

In a preferred embodiment is provided a method according to the invention wherein said chemical reaction comprises a hydrolysis of said radioactive compound, wherein said hydrolysis occurs during said heating, wherein said hydrolysis comprises a removal of a protecting group from said radioactive compound, wherein the yield of any side product of said removal of a protecting group is less than 15%, 14.5%, 14%, 13.5%, 13%, 12.5%, 12%, 11.5%, 11%, 10.5%, 10%, 9.5%, 9%, 8.5%, 8%, 7.5%, 7%, 6.5%, 6%, 5.5%, 5%, 4.8%, 4.6%, 4.4%, 4.2%, 4%, 3.8%, 3.6%, 3.4%, 3.2%, 3%, 2.8%, 2.6%, 2.4%, 2.2%, 2%, 1.8%, 1.6%, 1.4%, 1.2%, 1%, 0.8%, 0.6%, 0.4%, 0.2%, 0.1%. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In the context of deprotection, unless explicitly defined otherwise, "deprotection yield" and "deprotection reaction yield" refer to the ratio of the number of fully deprotected radioactive compounds to number of protected and partially deprotected radioactive compounds, whereas "yield of the fully deprotected radioactive compound" refers to the overall yield of the fully deprotected radioactive compound as defined above. In other words, if "deprotection" is used as a noun modifier of "yield", side reactions are not taken into account. If "deprotection" is not used as a noun modifier, reference is made to the yield as defined above.

In Examples 1 and 2, a method according to the invention wherein said chemical reaction comprises a deprotection of Boc₂-[¹³¹l]SGMIB, namely the removal of a Boc protecting group, during said heating. Under the conditions described therein, almost full deprotection was acquired (yield of the corresponding fully deprotected radioactive compound above 90%), whereas side products were only formed in a yield ranging from 3 to 12 %. These findings are exceptional and contrast with methods for deprotection disclosed in the art, as they typically report about a 30% yield of the fully deprotected product. In addition, the level of side products reported in the art for methods for deprotection is about 25%.

In a preferred embodiment is provided a method according to the invention wherein said chemical reaction comprises an **alkylation** of said radioactive compound, preferably wherein said alkylation occurs during said heating. An alkylation preferably means a nucleophilic substitution or addition reaction, wherein said radioactive compound may act either as a nucleophile or as an electrophile, resulting in the net addition of an alkyl group on said radioactive compound. Said alkyl group is preferably selected from the group consisting of branched, unbranched and cyclic C1-C8 alkyl groups, more preferably from the group consisting of methyl, ethyl, n-propyl, iso-propyl, c-propyl, n-butyl, sec-butyl, tert-butyl, iso-butyl and c-butyl. Said alkyl is optionally substituted. The product of an alkylation of a said radioactive compound is called an alkylated radioactive compound. Preferably, said alkylation does not comprise the use of an alkaline solution.

In a more preferred embodiment is provided a method according to the invention wherein said chemical reaction comprises an alkylation of said radioactive compound, wherein said alkylation occurs during said heating, wherein said alkylation has a high activation barrier, preferably wherein said solid phase is a facilitator for said alkylation.

In the preferred embodiments above, the degree of radiolysis during said alkylation is preferably lower than 5%, 4.5%, 4%, 3.5%, 3%, 2.5%, 2%, 1.5%, 1%, 0,9%, 0.8%, 0.7%, 0.6%, 0.5%, 0.4%, 0.3%, 0.2%, or 0.1%. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In the preferred embodiments above, the yield of said alkylated radioactive compound is at least 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 92.5%, 95%, or 97.5%. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In a most preferred embodiment is provided a method according to the invention wherein said chemical reaction comprises an alkylation of said radioactive compound, wherein said alkylation occurs during said heating, wherein the degree of radiolysis during said alkylation is lower than 5% and wherein the yield of said alkylated radioactive compound is at least 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 92.5%, 95%, or 97.5%, preferably wherein the degree of radiolysis during said alkylation is lower than 3% and wherein the yield of said alkylated radioactive compound is at least 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 92.5%, 95%, or 97.5%. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In a most preferred embodiment is provided a method according to the invention wherein said chemical reaction comprises an alkylation of said radioactive compound, wherein said alkylation occurs during said heating, wherein the degree of radiolysis during said alkylation is lower than 5% and wherein the yield of said alkylation product is at least 30%, preferably wherein the degree of radiolysis during said alkylation is lower than 3% and wherein the yield of said alkylation product is at least 35%, wherein the duration of said heating is in the range from 1 minute up to 10 minutes, more preferably in the range from 1 minute up to 5 minutes, and said heating of said mixture is to a temperature selected in the range from 30°C up to 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, 145°C, or 150°C. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes, or said heating of said mixture is to a temperature selected in the range from 30°C up to 55°C and the duration of said heating is selected in the range from 1 minute up to 5 minutes. Most preferably, said heating of said mixture is to a temperature selected in the range from 30°C up to 55°C and the duration of said heating is selected in the range from 1 minute up to 10 minutes.

In a most preferred embodiment is provided a method according to the invention wherein said chemical reaction comprises an alkylation of said radioactive compound, wherein said alkylation occurs during said heating, wherein the yield of said alkylation product is at least 30%, preferably wherein the yield of said alkylation product is at least 35%, wherein the duration of said heating is in the range from 1 minute up to 10 minutes, more preferably in the range from 1 minute up to 5 minutes, and said heating of said mixture is to a temperature selected in the range from 30°C up to 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, 145°C, or 150°C. Preferably, said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes, or said heating of said mixture is to a temperature selected in the range from 30°C up to 55°C and the duration of said heating is selected in the range from 1 minute up to 5 minutes. Most preferably, said heating of said mixture is to a temperature selected in the range from 30°C up to 55°C and the duration of said heating is selected in the range from 1 minute up to 10 minutes.

### Attachment to the solid phase

In a preferred embodiment is provided a method according to the invention, wherein contacting said mixture with said solid phase does not result in the attachment of said radioactive compound to said solid phase. In other words, neither chemisorption nor physisorption between said radioactive compound and said solid phase occurs as the result of said contacting. According to this embodiment, said radioactive compound reacts in the bulk of said mixture, i.e. without forming a bond with said solid phase. Preferably, said solid phase is a facilitator.

In another preferred embodiment is provided a method according to the invention, wherein contacting said mixture with said solid phase results in the **attachment** of said radioactive compound to said solid phase. Preferably, said radioactive compound is attached to said solid phase during step (b). In the context of this embodiment, said attaching preferably results in a non-covalent bond due to adsorption of said radioactive compound to said solid phase. In other words, according to this preferred embodiment, in case said solid phase enhances the rate of said chemical reaction of a radioactive compound, said solid phase acts as a facilitator instead of as a catalyst during step (b).

In an even more preferred embodiment is provided a method according to the invention, wherein (a) contacting said mixture with said solid phase results in the attachment of said radioactive compound to said solid phase, and wherein said radioactive compound remains attached to said solid phase at the end of step (b).

The attachment of the radioactive compound to said solid phase in a method according to the embodiment above has the advantage that the solid phase with the radioactive compound can be reacted further, minimizing the need for purification between reaction steps. For example, the deprotection of Boc₂-[¹³¹I]SGMIB via a method according to the invention as described in Examples 1 and 2 results in the attachment of fully deprotected SGMIB on Sep-Pak tC18 comprised in a standard SPE cartridge. This cartridge may be used for further reactions.

In this light, in a preferred embodiment is provided a method according to the invention, wherein (a) contacting said mixture with said solid phase results in the attachment of said radioactive compound to said solid phase, wherein said radioactive compound remains attached to said solid phase at the end of step (b), wherein said method comprises the step of (b') performing a further chemical reaction of said radioactive compound at the end of step (b). Preferably, no purification steps are needed between steps (b) and (b') comprised in a method according to the invention, as understood by the skilled person. Preferably, said radioactive compound remains attached to said solid phase at the end of step (b'). In this context, a further chemical reaction should be interpreted as a chemical reaction taking place after step (b), wherein performing said further chemical reaction is comprised in a method for performing a chemical reaction according to the invention. This is an advantage conferred by the method of the invention as further reactions could be directly carried out on the obtained radioactive compound still attached to the solid phase and therefore no purification is needed of this compound in order to carry out this further reaction.

During or at the end of a method according to the invention, said radioactive compound which is attached to the solid phase, should be able to be detached. For example, this could be necessary for performing a further chemical reaction which could not be performed while said radioactive compound is attached to said solid phase.

In a preferred embodiment is provided a method according to the invention, wherein (a) contacting said mixture with said solid phase results in the attachment of said radioactive compound to said solid phase, wherein said method further comprises the step of (c) detaching said radioactive compound from said solid phase. Preferably, step (c) is performed after step (b). More preferably, step (c) is performed after step (b').

In the context of this application, the attachment and the detachment of said radioactive compound should not be interpreted narrowly. If it is stipulated that contacting said mixture with said solid phase results in the attachment of said radioactive compound to said solid phase, and that said compound remains attached to said solid phase at the end of step (b) and/or at the end of step (b'), it should be understood that this encompasses that any (desired) radioactive product of said radioactive compound, including any (desired) radioactive intermediate, remains attached to said solid phase. For example, if (b) heating said mixture results in a deprotection of said radioactive compound, and it is stipulated that said radioactive compound remains attached to said solid phase at the end of step (b), it should be understood that the corresponding deprotected radioactive compound remains attached to said solid phase. Likewise, detaching said radioactive compound encompasses detaching any desired product of a chemical reaction, or a step thereof, which has occurred during (a) said contacting with said solid phase and/or (b) said heating of said mixture and/or (b') said performing a further chemical reaction. Preferably, said desired product is a radioactive compound.

In the context of the embodiments above, an **eluent** is defined as a liquid which is used to separate said mixture from said solid phase in step (c). In a more preferred embodiment is provided a method according to the invention, wherein (a) contacting said mixture with said solid phase results in the attachment of said radioactive compound to said solid phase, wherein said method comprises the step of (c) detaching said radioactive compound from said solid phase by contacting said solid phase with an eluent, wherein said eluent is selected from the group consisting of aqueous solutions, organic solvents or a mixture thereof, preferably wherein said organic solvent is a mixture of water and ethanol, more preferably wherein said organic solvent is ethanol. Preferably, step (c) is performed after step (b). More preferably, step (c) is performed after step (b').

A mixture of water and ethanol is preferably selected from the group consisting of 0.5wt%, 1wt%, 2wt%, 3wt%, 4wt%, 5wt%, 6wt%, 7wt%, 8wt%, 9wt%, 10wt%, 11wt%, 12wt%, 13wt%, 14wt%, 15wt%, 16wt%, 17wt%, 18wt%, 19wt%, 20wt%, 21wt%, 22wt%, 23wt%, 24wt%, 25wt%, 26wt%, 27wt%, 28wt%, 29wt%, 30wt%, 31wt%, 32wt%, 33wt%, 34wt%, 35wt%, 36wt%, 37wt%, 38wt%, 39wt%, 40wt%, 41wt%, 42wt%, 43wt%, 44wt%, 45wt%, 46wt%, 47wt%, 48wt%, 49wt%, 50wt%, 51wt%, 52wt%, 53wt%, 54wt%, 55wt%, 56wt%, 57wt%, 58wt%, 59wt%, 60wt%, 61wt%, 62wt%, 63wt%, 64wt%, 65wt%, 66wt%, 67wt%, 68wt%, 69wt%, 70wt%, 71wt%, 72wt%, 73wt%, 74wt%, 75wt%, 76wt%, 77wt%, 78wt%, 79wt%, 80wt%, 81wt%, 82wt%, 83wt%, 84wt%, 85wt%, 86wt%, 87wt%, 88wt%, 89wt%, 90wt%, 91wt%, 92wt%, 93wt%, 94wt%, 95wt%, 96wt%, 97wt%, 98wt%, 99wt%, or 99.5wt% of ethanol in water.

### Radioactive compound

In a method according to the invention, said radioactive compound does not comprise fluorine-18.

In a more preferred embodiment is provided a method according to the invention, wherein said radioactive compound comprises a radionuclide selected from the group consisting of a-emitters and β-emitters, preferably wherein said radionuclide is selected form the group consisting of hydrogen-3, astatine-211, carbon-11, carbon-14, bromine-76, iodine-123, iodine-124, iodine-125, iodine-131, phosphorus-32, and sulfur-35, most preferably wherein said radionuclide is selected from the group consisting of astatine-211, iodine-123, iodine-124 and iodine-131. The radionuclide used is not fluorine-18.

In a preferred embodiment is provided a method according to the invention, wherein said radioactive compound comprises a radionuclide which is both a β-emitter and a γ-emitter, preferably wherein said radionuclide is iodine-131.

In a preferred embodiment is provided a method according to the invention, wherein said radioactive compound comprises one, and not more than one, radionuclide, preferably wherein said radionuclide is iodine-131.

In a preferred embodiment is provided a method according to the invention, wherein the radionuclides comprised in said radioactive compound have an atom number from 11 up to 118, preferably form 19 up to 118, more preferably from 37 up to 118.

In a preferred embodiment, the feature "wherein said radioactive compound does not comprise fluorine-18" is replaced by "wherein any radionuclide comprised in said radioactive compound is selected from the group consisting of hydrogen-3, astatine-211, carbon-11, carbon-14, bromine-76, iodine-123, iodine-124, iodine-125, iodine-131, phosphorus-32, and sulfur-35". In other words, a method according to this preferred embodiment may be formulated as: a method for performing a chemical reaction of a radioactive compound comprised in a mixture, wherein said method comprises the following steps: (a) contacting said mixture with a solid phase, followed by (b) heating said mixture to a temperature selected in the range from 30°C up to 150°C, wherein steps (a) and (b) do not involve contacting said solid phase with an alkaline solution, wherein said chemical reaction does not result in the formation of a new bond on a radionuclide comprised in said radioactive compound, wherein any radionuclide comprised in said radioactive compound is selected from the group consisting of hydrogen-3, astatine-211, carbon-11, carbon-14, bromine-76, iodine-123, iodine-124, iodine-125, iodine-131, phosphorus-32, and sulfur-35.

In view of the above, a method according to the previous embodiment may thus be formulated as: a method for performing a chemical reaction of a radioactive compound comprised in a mixture, wherein said method comprises the following steps: (a) contacting said mixture with a solid phase, followed by (b) heating said mixture to a temperature selected in the range from 30°C up to 150°C, wherein during steps (a) and (b) any solution contacting said solid phase is a neutral or an acidic solution, wherein said chemical reaction does not result in the formation of a new bond on a radionuclide comprised in said radioactive compound, wherein any radionuclide comprised in said radioactive compound is selected from the group consisting of hydrogen-3, astatine-211, carbon-11, carbon-14, bromine-76, iodine-123, iodine-124, iodine-125, iodine-131, phosphorus-32, and sulfur-35.

In view of the above, a method according to the previous embodiment may thus be formulated as: a method for performing a chemical reaction of a radioactive compound comprised in a mixture, wherein said method comprises the following steps: (a) contacting said mixture with a solid phase, followed by (b) heating said mixture to a temperature selected in the range from 30°C up to 150°C, wherein steps (a) and (b) do not involve contacting said solid phase with an alkaline solution, wherein said chemical reaction is a post-labeling reaction, wherein any radionuclide comprised in said radioactive compound is selected from the group consisting of hydrogen-3, astatine-211, carbon-11, carbon-14, bromine-76, iodine-123, iodine-124, iodine-125, iodine-131, phosphorus-32, and sulfur-35.

In view of the above, a method according to the previous embodiment may thus be formulated as: a method for performing a chemical reaction of a radioactive compound comprised in a mixture, wherein said method comprises the following steps: (a) contacting said mixture with a solid phase, followed by (b) heating said mixture to a temperature selected in the range from 30°C up to 150°C, wherein during steps (a) and (b) any solution contacting said solid phase is a neutral or an acidic solution, wherein said chemical reaction is a post-labeling reaction, wherein any radionuclide comprised in said radioactive compound is selected from the group consisting of hydrogen-3, astatine-211, carbon-11, carbon-14, bromine-76, iodine-123, iodine-124, iodine-125, iodine-131, phosphorus-32, and sulfur-35.

In a preferred embodiment is provided a method according to the invention, wherein said radioactive compound is selected from the group consisting of N-succinimidyl-4-(1,2-bis(tert-butoxycarbonyl)guanidino)methyl-3-[¹³¹I]iodobenzoate (Boc₂-[¹³¹I]SGMIB), N-succinimidyl-4-(1-tert-butoxycarbonylguanidino)methyl-3-[¹³¹I]iodobenzoate (Boc-[¹³¹I]SGMIB), N-succinimidyl-3-(1,2-bis(tert-butoxycarbonyl)guanidino)methyl-3-[¹³¹I]iodobenzoate (iso-Boc₂-[¹³¹I]SGMIB), N-succinimidyl-3-(1-tert-butoxycarbonylguanidinomethyl-3-[¹³¹I]iodobenzoate (iso-Boc-[¹³¹I]SGMIB)), N,N'-bis(tert-butoxycarbonyl)-3-[¹²³I]iodobenzylguanidine (Boc₂-[¹³¹I]MIBG), N-tert-butoxycarbonyl-3-[¹²³I]iodobenzylguanidine (Boc-[¹³¹I]MIBG), (2R,3S)-2-[(2-[¹²³I]iodophenoxy)phenylmethyl]-N- tert-butoxycarbonyl-morpholine (Boc-[¹²³I]I-NKJ64) and (S)-2-N- tert-butoxycarbonyl -3-(4-[¹²⁵I]iodophenyl)propanoate (Boc-[¹²⁵I]I-Phe), preferably wherein said radioactive compound is N-succinimidyl-4-(1,2-bis(tert-butoxycarbonyl)guanidino)methyl-3-[¹³¹I]iodobenzoate (Boc2-[¹³¹I]SGMIB).

It is clear to the skilled person that the two tert-butoxycarbonyl (Boc or tert-Boc) groups comprised in Boc₂-[¹³¹I]SGMIB are protecting groups. In the context of the deprotection reactions defined above, the complete deprotection of Boc₂-[¹³¹I]SGMIB leads to N-succinimidyl-4-guanidinomethyl-3-[¹³¹I]iodobenzoate ([¹³¹I]SGMIB). The incomplete or partial deprotection of Boc₂-[¹³¹I]SGMIB leads to N-succinimidyl-4-(1-tert-butoxycarbonylguanidino)methyl-3-[¹³¹I]iodobenzoate (1'-Boc-[¹³¹I]SGMIB) or N-succinimidyl-4-(2-tert-butoxycarbonylguanidino)methyl-3- [¹³¹I]iodobenzoate (2'-Boc-[¹³¹I]SGMIB), both of which may be denoted by Boc-[¹³¹I]SGMIB.

In a more preferred embodiment is provided a method according to the invention, wherein said chemical reaction comprises a hydrolysis of said radioactive compound, wherein said hydrolysis occurs during said heating, wherein said hydrolysis comprises a removal of a protecting group from said radioactive compound, wherein said radioactive compound is N-succinimidyl-4-(1,2-bis(tert-butoxycarbonyl)guanidino)methyl-3-[¹³¹I]iodobenzoate (Boc₂-[¹³¹I]SGMIB),
- wherein the degree of radiolysis during said hydrolysis is lower than 5%, preferably lower than 3%; and/or
- wherein said hydrolysis occurs in an aqueous solution at a pH lower than 7, preferably lower than 6, preferably wherein said aqueous solution comprises phosphoric acid; and/or
- wherein the yield of N-succinimidyl-4-guanidinomethyl-3-[¹³¹I]iodobenzoate ([¹³¹I]SGMIB) is at least 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 92.5%, 95% or 97.5%; and/or
- wherein the duration of said heating is selected in the range from 1 minute up to 10 minutes. More preferably, the duration of said heating is selected in the range from 1 minute up to 5 minutes.

In a most preferred embodiment is provided a method according to the invention, wherein said chemical reaction comprises a hydrolysis of said radioactive compound, wherein said hydrolysis occurs during said heating, wherein said hydrolysis comprises a removal of a protecting group from said radioactive compound, wherein said radioactive compound is N-succinimidyl-4-(1,2-bis(tert-butoxycarbonyl)guanidino)methyl-3-[¹³¹I]iodobenzoate (Boc₂-[¹³¹I]SGMIB),
- wherein the degree of radiolysis during said hydrolysis is lower than 5%, preferably lower than 3%; and/or
- wherein said hydrolysis occurs in an aqueous solution at a pH lower than 7, preferably lower than 6, preferably wherein said aqueous solution comprises phosphoric acid; and/or
- wherein the yield of N-succinimidyl-4-guanidinomethyl-3-[¹³¹I]iodobenzoate ([¹³¹I]SGMIB) is at least 30%, preferably at least 35%; and/or
- wherein the duration of said heating is in the range from 1 minute up to 10 minutes, more preferably in the range from 1 minute up to 5 minutes; and/or
- wherein said heating of said mixture is to a temperature selected in the range from 30°C up to 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, 145°C, or 150°C, preferably wherein said heating of said mixture is to a temperature selected in the range from 30°C up to 55°C.

In an even more preferred embodiment is provided a method according to the previous embodiment, wherein contacting said mixture with said solid phase results in the attachment of said radioactive compound to said solid phase, and wherein said method further comprises the step of (c) detaching said radioactive compound from said solid phase using an eluent, wherein said eluent is selected from the group consisting of aqueous solutions, organic solvents or a mixtures thereof, preferably wherein said solvent is a mixture of water and ethanol, more preferably wherein said solvent is ethanol. In these preferred embodiments, said solid phase is preferably a facilitator.

In a preferred embodiment is provided a method according to the invention, wherein said chemical reaction comprises a hydrolysis of said radioactive compound, wherein said hydrolysis occurs during said heating, wherein said hydrolysis comprises a removal of a protecting group from said radioactive compound, wherein said radioactive compound is N-succinimidyl-4-(1,2-bis(tert-butoxycarbonyl)guanidino)methyl-3-[¹³¹I]iodobenzoate (Boc₂-[¹³¹I]SGMIB), wherein N-succinimidyl-4-(1,2-bis(tert-butoxycarbonyl)guanidino)methyl-3-[(131)I]iodobenzoate (Boc₂-[¹³¹I]SGMIB) is converted to N-succinimidyl-4-guanidinomethyl-3-[(131)I]iodobenzoate ([¹³¹I]SGMIB) with a yield of at least 30% during heating, as determined by quantitative HPLC, wherein said heating of said mixture is to a temperature selected in the range from 30°C up to 55°C. Preferably, the duration of said heating is selected in the range from 1 minute up to 5 or 10 minutes. More preferably, said heating of said mixture is to a temperature selected in the range from 30°C up to 55°C and the duration of said heating is selected in the range from 1 minute up to 10 minutes.

In a preferred embodiment is provided a method according to the invention, wherein the activity of said radioactive compound is from 0.1 Curie up to 100 Curie, from 0.5 Curie up to 100 Curie, from 1 Curie up to 100 Curie, from 2 Curie up to 100 Curie, from 3 Curie up to 100 Curie, from 4 Curie up to 100 Curie, from 5 Curie up to 100 Curie, from 10 Curie up to 100 Curie. Preferably, said activity of said radioactive compound is measured at the end of step (b) comprised in a method according to the invention.

### Solid phase and architecture

In a preferred embodiment is provided a method according to the invention, wherein said solid phase is a silica or a polymeric solid phase, preferably selected from the group consisting of Sep-Pak tC18, Step-Pak C18, Oasis HLB, Oasis MCX, Oasis MAX and Sephadex LH-20, preferably wherein said solid phase is Sep-Pak tC18.

In a preferred embodiment is provided a method according to the invention, wherein said solid phase is a silica or a polymeric solid phase, wherein said solid phase is a facilitator for said reaction.

A (chemical) reaction wherein a solid phase acts as a facilitator is called a (chemical) reaction facilitated by said solid phase in the context of this application.

In a preferred embodiment is provided a method according to the invention wherein said solid phase and said mixture are comprised in a cartridge, preferably a solid phase extraction (SPE) cartridge. It is well-known to the skilled person that SPE cartridges comprising the above-specified solid phases are commercially available.

In a more preferred embodiment is provided a method according to the invention, wherein said solid phase and said mixture are comprised in a cartridge, preferably a solid phase extraction (SPE) cartridge, wherein said cartridge is discardable, preferably wherein said cartridge is single-use. A discardable cartridge is defined as a cartridge which may only be used in a limited number of iterations of a method according to the invention, wherein said limited number is preferably 100, 50, 20, 15, 10, 9, 8, 7, 6, 5, 4, 3, 2 or 1. Without being bound to this theory, using a discardable cartridge for more iterations may lead to lower yields of the desired product of said chemical reaction according to the invention.

In an even more preferred embodiment is provided a method according to the invention, wherein said solid phase and said mixture are comprised in a cartridge, preferably a solid phase extraction (SPE) cartridge, wherein said cartridge is single-use. A single-use cartridge is defined as a cartridge which may only be used in a single iteration of a method according to the invention, as explained above.

In a preferred embodiment is provided a method according to the invention, wherein said method is automated. A method for performing a chemical reaction is automated in the context of this invention if (a) said contacting with said solid phase and/or (b) said heating of said mixture and/or (c) said detaching of said radioactive compound and/or (d) said attaching a biological moiety do not require direct manipulation by a human operator. Preferably, direct manipulation means handling or holding a recipient comprising a mixture comprising said radioactive compound, and/or manually adjusting the reaction conditions such as temperature during said (a) and/or (b) and/or (c) and/or (d).

A method according to the invention preferably comprises the use of a device or a system according to the invention, as defined below, wherein said (b) heating said mixture during said method is achieved by said powering of said heating means comprised in said device or said system. Preferably, a method according to the invention is a method performed with a system according to the invention, as defined in the context of the fifth aspect of the current invention.

It is clear for the skilled person that, whenever a device according to the invention or a system according to the invention is used in a method according to the invention, as described here or in other disclosures in the context of this application:
- said mixture, mentioned in the context of a device or a system according to the invention, is said mixture comprising a radioactive compound, mentioned in the context of said method according to the invention;
- said (b) heating said mixture during said method is achieved by said powering of said heating means comprised in said device or said system;
- preferably said chemical reaction, as defined in the context of a method according to the invention, takes place in said chemical reaction vessel comprised in said system;
- preferably said solid phase in step (a) of said method is said solid phase comprised in said system.

In this light, all preferences and embodiments disclosed in the context of a method according to the invention may be applied mutatis mutandis to a device or a system according to the invention. Moreover, all preferred embodiments and definitions relating to a device according to the invention or a system according to the invention should also be interpreted as preferred embodiments and definitions relating to a method according to the invention comprising the use of such a device or system.

Specifically, as one example, in a preferred embodiment is provided a method according to the invention wherein said method comprises the use of a system or device according to the invention, wherein the chemical reaction vessel comprised in said system is an SPE cartridge.

### Biological moieties

In a preferred embodiment is provided a method according to the invention, wherein contacting said mixture with said solid phase results in the attachment of said radioactive compound to said solid phase, wherein said chemical reaction comprises a hydrolysis of said radioactive compound, wherein said hydrolysis occurs during said heating, wherein said hydrolysis comprises a removal of a protecting group from said radioactive compound, wherein said method further comprises the step of (c) detaching said radioactive compound from said solid phase, wherein said method comprises (d) attaching a biological moiety to the deprotected radioactive compound obtained at the end of step (c), wherein said attaching results in a radiolabeled biological moiety. In the context of this application, a method according to this embodiment is called a method for attaching a biological moiety according to the invention. A biological moiety is preferably a lipid, a polymer of monosaccharides or a polymer of amino acids, more preferably a polymer of amino acids.

In a preferred embodiment is provided a method for attaching a biological moiety according to the invention, wherein said biological moiety is a peptide, a protein scaffold, or an antibody or a fragment thereof, preferably wherein said biological moiety is a diagnostic and/or a therapeutic compound.

A preferred peptide, which may be used as a biological moiety in the context of this application, is selected from the group consisting of FAPI, PSMA-617, PSMA-11 Substance P, somatostatin analogues, gastric releasing peptide receptor ligands, and ECL1i.

A preferred protein scaffold, which may be used as a biological moiety in the context of this application, is selected from the group consisting of Affibody, including ZHER2:342 and ABY-025, Affitin, knottin, and Darpin.

In a preferred embodiment is provided a method for attaching a biological moiety according to the invention, wherein said biological moiety is an antibody or a fragment thereof, preferably wherein said antibody or fragment thereof is a diagnostic and/or a therapeutic compound.

An antibody refers to polyclonal antibodies, monoclonal antibodies, humanized antibodies, single-chain antibodies, and fragments thereof such as Fab, F(ab)2, Fv, Fab, F(ab)2, scFv, minibody, biabody, scFv-Fc, and other fragments that retain the antigen binding function of the parent antibody. As such, an antibody may refer to an immunoglobulin or glycoprotein, or fragment or portion thereof, or to a construct comprising an antigen-binding portion comprised within a modified immunoglobulin-like framework, or to an antigen-binding portion comprised within a construct comprising a non- immunoglobulin-like framework or scaffold.

A preferred antibody or a fragment thereof, which may be used as a biological moiety in the context of this application, is selected from the group consisting of FF-21101, h11B6, CYT-500, chimeric G250, B-B4, Mu11-1F4, BC-8, trastuzumab, pertuzumab, cetuximab, ibritumomab, tositumomab, rituximab, girentuximab, lintuzumab, F16SIP, huA33, J591, 81C6, CC49-deltaCH2, VRC01, Hu3S193, A33, MN-14, B3, BW250/183, Lym-1, TNT-1/B, 8H9, Diabody T84.66, huLL2, chimeric T84.66, MAB-145, huM195, CC49, daclizumab, hPAM4, BU-12, 3F8, amatuximab, Atezolizumab, omburtamab, IAB22M2X CD8 minibody, FPI-1434, BC8-B10, F(ab)2 MX35, 9.2.27, epratutumab, BAY 861903, and BAY 2315158.

A monoclonal antibody refers to an antibody composition having a homogeneous antibody population. The term is not limited regarding the species or source of the antibody, nor is it intended to be limited by the manner in which it is made. The term encompasses whole immunoglobulins as well as fragments and others that retain the antigen binding function of the antibody. Polyclonal antibody refers to an antibody composition having a heterogeneous antibody population.

A therapeutic compound is a compound for treating, ameliorating, preventing, and/or delaying a disease or a condition. A diagnostic compound is a compound for detecting a disease or a condition in an individual. In this context, said antibody or fragment thereof is able to bind an antigen which is associated with a disease or a condition. Preferably, said antigen is expressed in a cell associated with said disease or condition.

Preferably, said disease is cancer, more preferably a solid tumor, most preferably a solid tumor is selected from the group consisting of breast cancer, ovarian cancer, gastric cancer, multiple myeloma and lymphoma. Alternatively, said cancer may be a hematological cancer.

In a preferred embodiment is provided a method for attaching a biological moiety according to the invention, wherein said biological moiety is an antibody or a fragment thereof, wherein said antibody or fragment thereof is a diagnostic and/or a therapeutic compound, wherein said diagnostic and/or therapeutic compound is targeted against an antigen expressed in a cell and/or present on the surface of a cell.

Antigens expressed in a tumor cell are tumor-specific antigens or cancer cell-specific antigens occurring specifically or being expressed specifically and/or abundantly in or on the surface of tumor cells or cancer cells, respectively, and, preferably, not or only in relatively low concentration or density in or on the surface of normal healthy cells. When these tumor-specific or cancer cell-specific antigens are bound an antigen or a VHH or a fragment thereof used as a biological moiety in a method for attaching a biological moiety according to the invention, the corresponding tumor or cancer cells onto which the antigens are expressed may be killed or at least arrested, inhibited or reduced in their growth through the mechanism of radiotoxicity, or the corresponding tumor or cancer cells onto which the antigens are expressed may be radiolabeled for diagnostic purposes.

Suitable tumor-specific or cancer cell-specific target molecules are readily available from existing literature or patent databases for the skilled person and include, without limitation any protein produced in a tumor cell that has an abnormal structure due to mutation, including the abnormal products of ras and p53 genes, tissue differentiation antigens, mutant protein antigens, oncogenic viral antigens, cancer-testis antigens, oncofetal antigens and vascular or stromal specific antigens. Examples of specific tumor antigens include but are not limited to CTAG1B, MAGEA1, the enzyme tyrosinase, alphafetoprotein (AFP), carcinoembryonic antigen (CEA), EBV and HPV, abnormally structured cell surface glycolipids and glycoproteins, HER2, EGFR, PSMA, FAP, CD33, CD45, tenacin-C, IGF-1 R, SSR2, and variants thereof.

Hence, a method for attaching a biological moiety according to the invention wherein said biological moiety is an antibody or a fragment thereof, wherein said antibody or fragment thereof is a diagnostic and/or a therapeutic compound, wherein said diagnostic and/or therapeutic compound is targeted against an antigen expressed in a tumor cell and/or present on the surface of a tumor cell, provides a method to obtain a radiolabeled diagnostic and/or therapeutic compound against a tumor cell.

In the embodiments above, said antigen is preferably HER2, and said tumor cell is thus a HER2 positive tumor cell.

In other words, in some embodiments, the product of a method for attaching a biological moiety according to the invention, is capable of killing a tumor cell or a cancer cell that expresses an antigen against which an antibody, a VHH or fragment thereof is used as a biological moiety in said method, or can reduce or slow the growth and/or proliferation of such a tumor cell or cancer cell.

In some embodiments, an antibody, VHH or fragment thereof used as a biological moiety in a method for attaching a biological moiety according to the invention is monovalent. Herein, monovalent means that said antibody, VHH or fragment thereof contains only one binding site for a given antigen, as defined above.

An antigen expressed in a tumor cell is preferably HER2. Therefore, in some embodiments, the antibody, VHH or fragment thereof used in a method for attaching a biological moiety according to the invention specifically binds to HER2.

In a preferred embodiment is provided a method according to the previous embodiment, wherein said antibody or fragment thereof is a heavy chain variable domain derived from a heavy chain antibody (VHH), or a fragment thereof, preferably wherein said heavy chain antibody (VHH), or a fragment thereof, has at least 80% amino acid identity with or has an amino acid sequence SEQ ID NO: 7 or SEQ ID NO: 8.

A fragment of an antibody may be a heavy chain variable domain of an antibody or a fragment thereof. A heavy chain variable domain of an antibody or a fragment thereof, as used herein, means (i) the variable domain of the heavy chain of a heavy chain antibody, which is naturally devoid of light chains (indicated herein as VHH) including but not limited to the variable domain of the heavy chain of heavy chain antibodies of camelids or sharks or (ii) the variable domain of the heavy chain of a conventional four-chain antibody or any fragments thereof, such as but not limited to one or more stretches of amino acid residues (i.e. small peptides) that are particularly suited for binding to a tumor antigen or an antigen present on cancer cells and which are present in, and/or may be incorporated into, the VHH as disclosed herein (or may be based on and/or derived from CDR sequences of the VHH as disclosed herein). Preferably, a heavy chain variable domain of an antibody or a fragment thereof is a VHH or a fragment thereof.

The amino acid sequence and structure of a heavy chain variable domain of an antibody can be considered, without however being limited thereto, to be comprised of four framework regions or "PR's", which are referred to in the art and hereinbelow as "framework region 1" or "FR1"; as "framework region 2" or "FR2"; as "framework region 3" or "FR3"; and as "framework region 4" or "FR4," respectively, which framework regions are interrupted by three complementary determining regions or "CDR's", which are referred to in the art as "complementarity determining region 1" or "CDR1"; as "complementarity determining region 2" or "CDR2"; and as "complementarity determining region 3" or "CDR3", respectively.

As used herein, the terms "complementarity determining region" or "CDR" within the context of antibodies refer to variable regions of either the H (heavy) or the L (light) chains (also abbreviated as VH and VL, respectively) and contain the amino acid sequences capable of specifically binding to antigenic targets. These CDR regions account for the basic specificity of the antibody for a particular antigenic determinant structure. Such regions are also referred to as "hypervariable regions". The CDRs represent non-contiguous stretches of amino acids within the variable regions but, regardless of species, the positional locations of these critical amino acid sequences within the variable heavy and light chain regions have been found to have similar locations within the amino acid sequences of the variable chains. The variable heavy and light chains of all canonical antibodies each have 3 CDR regions, each non-contiguous with the others (termed L1, L2, L3, H1, H2, H3) forthe respective light (L) and heavy (H) chains.

In some embodiments, as also further described hereinbelow, the total number of amino acid residues in a heavy chain variable domain of an antibody (including a VHH) can be in the region of 110-130, is preferably 112-115, and is most preferably 113. It should however be noted that parts, fragments or analogs of a heavy chain variable domain of an antibody are not particularly limited as to their length and/or size, as long as such parts, fragments or analogs retain (at least part of) the functional activity, and/or retain (at least part of) the binding specificity of the original a heavy chain variable domain of an antibody from which these parts, fragments or analogs are derived from. Parts, fragments or analogs retaining (at least part of) the functional activity, and/or retaining (at least part of) the binding specificity of the original heavy chain variable domain of an antibody from which these parts, fragments or analogs are derived from are also further referred to herein as "functional fragments" of a heavy chain variable domain. Preferably, a fragment of a heavy chain variable domain or of a VHH is functional fragment of said heavy chain variable domain or of said VHH, respectively.

The disclosure provides a number of stretches of amino acid residues that are particularly suited for binding to a tumor antigen or a cancer cell antigen, such as but not limited to HER2. These stretches of amino acid residues may be present in, and/or may be incorporated into, a VHH used as a biological moiety in a method for attaching a biological moiety according to the invention, in particular in such a way that they form (part of) the antigen binding site of that VHH. As these stretches of amino acid residues were first generated as CDR sequences of antibodies (or may be based on and/or derived from such CDR sequences), they will also generally be referred to herein as 'CDR sequences' (e.g. as CDR1 sequences, CDR2 sequences and CDR3 sequences, respectively).

Thus, in particular, but non-limiting embodiments, a VHH used as biological moiety in a method for attaching a biological moiety according to the invention comprises at least one amino acid sequence that is chosen from the group consisting of the CDR1 sequences, CDR2 sequences and CDR3 sequences that are described herein. In some embodiments said VHH may comprise at least one antigen binding site, wherein said antigen binding site comprises at least one combination of a CDR1 sequence, a CDR2 sequence and a CDR3 sequence that are described herein.

A VHH or a fragment thereof used a biological moiety in a method for attaching a biological moiety according to the invention having one of these CDR sequence combinations is preferably able to specifically bind (as defined herein) to a tumor- specific antigen and/or to a cancer-cell-specific antigen. In some embodiments, said VHH or fragment thereof specifically binds to a tumor-specific antigen and/or to a cancer-cell-specific antigen with dissociation constant (KD) of 10⁻⁸ M or less of said variable domain in solution. In particular embodiments, said VHH or fragment thereof is such that it can specifically bind to HER2 with a dissociation constant (KD) of less than 5 nM, such as from 1 up to 5 nM, preferably from 2 up to 3 nM.

Specific binding of a VHH to a tumor antigen or cancer cell antigen can be determined in any suitable manner known, including, for example biopanning, Scatchard analysis and/or competitive binding assays, such as radioimmunoassays (RIA), enzyme immunoassays (EIA) and sandwich competition assays, and the different variants thereof known in the art.

In further particular embodiments, a VHH or fragment thereof used as a biological moiety in a method for attaching a biological moiety according to the invention comprises at least one combination of CDR sequences chosen from the group comprising:
- a CDR1 region having SEQ ID NO: 1, a CDR2 region having has SEQ ID NO: 2, and a CDR3 region having SEQ ID NO: 3, and/or
- a CDR1 region having SEQ ID NO: 4, a CDR2 region having has SEQ ID NO: 5, and a CDR3 region having SEQ ID NO: 6.

SEQ ID NOs: 7 and 8 give the amino acid sequences of exemplary heavy chain variable domains that have been raised against HER2.

In some embodiments, a VHH used as a biological moiety in a method according to the invention is directed against a tumor-specific or cancer cell-specific target antigen, and has at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with at least one of the heavy chain variable domains of SEQ ID NOs: 7 or 8, or fragments thereof. In some embodiments, said VHH or fragment thereof directed against a tumor-specific or cancer cell-specific target antigen is encoded by a nucleic acid that which has at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with a nucleic acid sequence that encodes such heavy chain variable domains or fragments thereof.

In some embodiments, heavy chain variable domain sequences as disclosed herein are those which can bind to and/or are directed against HER2 and which have at least 90% amino acid identity with at least one of the heavy chain variable domains of SEQ ID NOs: 7 or 8, in which forthe purposes of determining the degree of amino acid identity, the amino acid residues that form the CDR sequences are disregarded.

In some embodiments, a VHH used as a biological moiety in a method according to the invention is directed against a tumor-specific or cancer cell-specific target antigen, wherein said VHH comprises an amino acid sequence selected from SEQ ID NOs: 9 to 13.

In some embodiments, a VHH used as a biological moiety in a method according to the invention is directed against a tumor-specific or cancer cell-specific target antigen, and has at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with at least one of the heavy chain variable domains of SEQ ID NOs: 9 to 13, or fragments thereof. In some embodiments, said VHH or fragment thereof directed against a tumor-specific or cancer cell-specific target antigen is encoded by a nucleic acid that which has at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with a nucleic acid sequence that encodes such heavy chain variable domains or fragments thereof.

In some embodiments, heavy chain variable domain sequences as disclosed herein are those which can bind to and/or are directed against HER2 and which have at least 90% amino acid identity with at least one of the heavy chain variable domains of SEQ ID NOs: 9 to 13, in which forthe purposes of determining the degree of amino acid identity, the amino acid residues that form the CDR sequences are disregarded.

In some embodiments, the radiolabeled VHH domains or fragments thereof used as a biological moiety in a method for attaching a biological moiety according to the invention may be optionally linked to one or more further groups, moieties, or residues via one or more linkers. These one or more further groups, moieties or residues can serve for binding to other targets of interest. It should be clear that such further groups, residues, moieties and/or binding sites may or may not provide further functionality to the heavy chain variable domains as disclosed herein and may or may not modify the properties of the heavy chain variable domain as disclosed herein. Such groups, residues, moieties or binding units may also for example be chemical groups which can be biologically active.

These groups, moieties or residues are, in some embodiments, linked N- or C- terminally to the heavy chain variable domain, in particularly C-terminally linked.

In some embodiments, the VHH domains or fragments thereof used as a biological moiety in a method for attaching a biological moiety according to the invention may also be chemically modified. For example, such a modification may involve the introduction or linkage of one or more functional groups, residues or moieties into or onto the heavy chain variable domain. These groups, residues or moieties may confer one or more desired properties or functionalities to the heavy chain variable domain. Examples of such functional groups will be clear to the skilled person.

For example, the introduction or linkage of such functional groups to a heavy chain variable domain can result in an increase in the solubility and/or the stability of the heavy chain variable domain, in a reduction of the toxicity of the heavy chain variable domain, or in the elimination or attenuation of any undesirable side effects of the heavy chain variable domain, and/or in other advantageous properties.

In particular embodiments, the one or more groups, residues, moieties are linked to the heavy chain variable domain via one or more suitable linkers or spacers.

In some embodiments, the one or more groups, residues or moieties do not confer to the radiolabelled VHH or fragments thereof as disclosed herein an extended half-life. Accordingly, in some embodiments, the radiolabelled VHH or fragments thereof used as a biological moiety in a method for attaching a biological moiety according to the invention are non-lifetime extended.

While the radiolabeled VHH domains specifically binding to a tumor- specific antigen and/or a cancer cell-specific antigen used as a biological moiety in a method for attaching a biological moiety according to the invention are generally in monomeric form, in particular alternative embodiments, two or more of the radiolabeled VHHs or fragments thereof as described above may be linked to each other or may be interconnected. In particular embodiments, the two or more VHH or fragments thereof are linked to each other via one or more suitable linkers or spacers. Suitable spacers or linkers for use in the coupling of different VHHs will be clear to the skilled person and may generally be any linker or spacer used in the art to link peptides and/or proteins.

Some exemplary suitable linkers or spacers include, but are not limited to, polypeptide linkers such as glycine linkers, serine linkers, mixed glycine/serine linkers, glycine- and serine-rich linkers or linkers composed of largely polar polypeptide fragments, or homo- or heterobifunctional chemical crosslinking compounds such as glutaraldehyde or, optionally PEG- spaced, maleimides or NHS esters.

### Method for performing a chemical reaction

As explained above, a reaction according to the invention is a post-labeling reaction of a radioactive compound. By definition, said chemical reaction does not result in the formation of a new bond on a radionuclide comprised in said radioactive compound. Nevertheless, a reaction according to the invention may be comprised in a broader chemical reaction, wherein said radioactive compound is formed as an intermediate, and wherein said broader reaction comprises a reaction wherein a new bond is formed on a radionuclide.

In a second aspect, the invention therefore provides a method for obtaining a radioactive product, wherein said method comprises:
(i) generating a radioactive compound comprised in a mixture; followed by
(ii) performing a post-labeling chemical reaction of said radioactive compound comprised in said mixture, wherein a method according to the invention is used to perform said post-labeling chemical reaction, wherein said radioactive product is a product of said post-labeling chemical reaction.

In the context of this second aspect of the invention, the same definitions and clarifications apply as defined above in the context of the first aspect of the invention, unless explicitly stated otherwise. Furthermore, all preferred embodiments and features of a method according to the invention, as disclosed above, may be applied to step (ii) of a method according to this second aspect mutatis mutandis.

In a preferred embodiment is provided a method according to this second aspect, wherein step (i) is performing a chemical reaction between a precursor and a radioactive component, wherein said precursor does not comprise a radionuclide.

Preferably, a precursor is an organic compound, more preferably wherein said precursor is N-succinimidyl 4-[1,2-bis(tert-butyloxycarbonyl)guanidinomethyl]-3-(trimethyl-stannyl)benzoate (Boc₂-SGMTB) or N-succinimidyl 3-[1,2-bis(tert-butyloxycarbonyl)guanidinomethyl]-5-(trimethyl-stannyl)benzoate (iso-Boc₂-SGMTB), most preferably wherein said precursor is N-succinimidyl 4-[1,2-bis(tert-butyloxycarbonyl)guanidinomethyl]-3-(trimethyl-stannyl)benzoate (BOC₂-SG MTB).

Preferably, a radioactive component is an inorganic compound comprising a radionuclide, more preferably an inorganic salt of a radionuclide or a binary acid of a radionuclide, even more preferably a sodium salt or a binary acid, most preferably said radioactive component is Na¹³¹I.

In a preferred embodiment is provided a method according to this second aspect, wherein said method is automated.

### Heating device

In a third aspect, the invention provides a device (1) for receiving and heating a chemical reaction vessel (3) comprising a mixture, said device (1) comprising heating means (5) and an opening (2) configured for receiving said chemical reaction vessel (3), said heating means (5) at least partially surrounding said opening; wherein said heating means comprises:
- an insulator polymer (9),
- a resistive conductor (8) embedded in said insulator polymer;
wherein said device (1) is configured for, when said chemical reaction vessel (3) comprising said mixture is present in said opening (2), heating the mixture present in said chemical reaction vessel (3) according to a predetermined temperature requirement by powering said heating means (5); preferably wherein said device (1) is a tubular sleeve and said opening is a lumen surrounded by said device (1). A device according to this third aspect of the invention is referred to as **a device according to the invention** in the current application.

Preferably, a method according to the invention comprises the use of a device according to the invention or is carried out on said device. Herein, said mixture, mentioned in the context of a device according to the invention, is said mixture comprising a radioactive compound, mentioned in the context of a method according to the invention; wherein said radioactive compound does not comprise fluorine-18, the heating of said mixture is to a temperature selected in the range from 30°C up to 150°C; and said step (b) of heating said mixture during said method is achieved by said powering of said heating means comprised in said device. Said chemical reaction, as defined in the context of a method according to the invention, preferably takes place in said chemical reaction vessel. In this light, all embodiments and preferred embodiments disclosed in the context of a method according to the invention may be applied mutatis mutandis to a device according to the invention.

A device according to the invention provides surface heating, which is advantageous in that it allows optimum control of the heating process, while being non-intrusive. It effectively removes the need to consider sensitive materials or critical maximum temperatures. It avoids problems with hot-spots or localized overheating. Additionally, it is non-intrusive, avoiding the practicalities of the introduction and cleaning of an intrusive heating device. Additionally, surface heating using polymer insulators, and especially in embodiments with a flexible silicone, leads to a lightweight and compact device, and has the advantage that it can be created solely for the given chemical reaction vessel and/or the given chemical reaction. Additionally, surface heating using polymer insulators provides low thermal mass, advantageous electrical insulation properties and robust construction, allowing high power densities and offering fast response to temperature control. Furthermore, a heating device according to the invention is simple, and therefore enables easy adherence to a predetermined temperature requirement.

In a fourth aspect, the invention provides a system (10) for performing a chemical reaction in a mixture, comprising:
- a device (1) according to the invention;
- a chemical reaction vessel (3) placed within the opening (2) of said device (1), said chemical reaction vessel (3) comprising a reactor chamber (30), an inlet (31), a housing, and a solid phase preferably suitable for acting as a facilitator of said chemical reaction;
wherein said system (10) is configured for, when said mixture is inserted in the reactor chamber (30) through said inlet (31), heating said mixture present in said chemical reaction vessel (3) according to a predetermined temperature requirement by powering said heating means (5), thereby allowing said chemical reaction to take place within said reaction chamber (30). A system according to this fourth aspect of the invention is referred to as a **system according to the invention** in the current application.

It is clear to the skilled person that the major difference between a device according to the invention and a system according to the system is the inclusion of the chemical reaction vessel. Hence, a device according to the invention can be envisioned as a system according to the invention without a chemical reaction vessel placed within the opening of said device.

Preferably, a method according to the invention to the invention comprises the use of a system according to the invention. Herein, said mixture, mentioned in the context of a system according to the invention, is said mixture comprising a radioactive compound, mentioned in the context of a method according to the invention, wherein said radioactive compound does not comprise fluorine-18; and the heating of said mixture is to a temperature selected in the range from 30°C up to 150°C; said solid phase comprised in said chemical reaction vessel is said solid phase in step (a) of said method according to the invention; and said (b) heating said mixture during said method is achieved by said powering of said heating means comprised in said device or said system. In other words, according to this preferred embodiment, said chemical reaction is a chemical reaction according to the invention. In this light, all embodiments and preferred embodiments disclosed in the context of a method according to the invention may be applied mutatis mutandis to a system according to the invention.

In a fifth aspect, the invention provides a method for performing a chemical reaction in a mixture, said method comprising the steps of:
- providing a system for performing a chemical reaction in a mixture, said system comprising:
   ∘ a device (1) for receiving and heating a chemical reaction vessel (3) comprising a mixture, said device (1) comprising a heating means (5) and an opening (2) configured for receiving said reaction vessel (3), said heating means (5) at least partially surrounding said opening;
   ∘ a chemical reaction vessel (3) placed within said opening (2), said chemical reaction vessel (3) comprising a reactor chamber (30), an inlet (31), a housing (33), and a solid phase preferably suitable for acting as a facilitator in said chemical reaction;
- inserting the mixture in said reactor chamber (3) via said inlet (31);
- heating the mixture heating present in the chemical reaction vessel (3) according to a predetermined temperature requirement by powering said heating means (5), thereby allowing said chemical reaction to take place within said reaction chamber (30).

Preferably, said system for performing a chemical reaction in a mixture according to the previous embodiment is a system according to the invention. In the context of this application, a method according to this preferred definition is called a **method performed with a system according to the invention.** More preferably, a method performed with a system according to the invention is a method according to the invention. In other words, a system according to the invention is more preferably used to perform a chemical reaction according to the invention.

The embodiments and preferred embodiments below relate to a device according to the invention, a system according to the invention, and/or a method performed with a system according to the invention.

In the context of the invention, a predetermined temperature requirement may relate to any set of parameters defining a thermal conditioning of the chemical reaction vessel and its contents. Particularly, the predetermined temperature requirement may comprise a maximum temperature and/or a time window during which a temperature between a minimal temperature and a maximal temperature is to be maintained and/or a maximal time for carrying out the heating and/or a minimal or maximal time for letting the chemical reaction take place.

In example embodiments, the predetermined temperature requirement may relate to requiring a target temperature of said mixture of any temperature in the range from 30°C up to 250°C, from 30°C up to 250°C, from 30°C up to 240°C, from 30°C up to 230°C, from 30°C up to 220°C, from 30°C up to 210°C, from 30°C up to 200°C, from 30°C up to 190°C, from 30°C up to 180°C, from 30°C up to 170°C, from 30°C up to 160°C, from 30°C up to 150°C, from 30°C up to 140°C, from 30°C up to 130°C, from 30°C up to 120°C, from 30°C up to 110°C, from 30°C up to 100°C, from 30°C up to 90°C, from 30°C up to 80°C, from 30°C up to 70°C, from 30°C up to 60°C, from 30°C up to 50°C, preferably for any duration between five seconds and one hour, e.g. three minutes, while starting from an initial temperature that is, e.g., 30 °C or 40 °C or 50 °C or 60 °C or 70 °C. The predetermined temperature requirement may furthermore specify one or more parameters regarding the transition from the initial temperature to the target temperature, e.g. a maximum time for the transition and/or the requirement that the overshoot remains below 5 °C or preferably below 1 °C.

Preferably, said target temperature of said mixture is selected in the range from 30°C up to 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, 145°C, or 150°C. Preferably, said target temperature of said mixture is selected in the range from 30°C, 35°C, 40°C, 45°C, 50°C, 55°C, 60°C, or 65°C up to 70°C. Preferably, said target temperature of said mixture is selected in the range from 30°C, 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, or 145°C up to 150°C.

Preferably, said target temperature of said mixture is selected in the range from 10°C centered around 35°C, 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, 140°C, or 145°C. Preferably, said target temperature of said mixture is selected in the range from 20°C centered around 40°C, 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, or 140°C. Preferably, said target temperature of said mixture is selected in the range of 30°C centered around 45°C, 50°C, 55°C, 60°C, 65°C, 70°C, 75°C, 80°C, 85°C, 90°C, 95°C, 100°C, 105°C, 110°C, 115°C, 120°C, 125°C, 130°C, or 135°C. Preferably, said target temperature of said mixture is selected in the range of 40°C centered around 50°C, 55°C, 60°C, 65°C, 70°C, 75°C, 80°C, 85°C, 90°C, 95°C, 100°C, 105°C, 110°C, 115°C, 120°C, 125°C, or 130°C. Preferably, said target temperature of said mixture is selected in the range of 50°C centered around 55°C, 60°C, 65°C, 70°C, 75°C, 80°C, 85°C, 90°C, 95°C, 100°C, 105°C, 110°C, 115°C, 120°C, or125°C.

Preferably, said predetermined temperature requirement means requiring a target temperature of said mixture selected in the range from 30°C up to 70°C for a duration selected in the range from 1 minute up to 15 minutes, or a target temperature selected in the range from 30°C up to 55°C for a duration selected in the range from 1 minute up to 15 minutes, or a target temperature selected in the range from 30°C up to 70°C for a duration selected in the range from 1 minute up to 10 minutes, or a target temperature selected in the range from 30°C up to 55°C for a duration selected in the range from 1 minute up to 10 minutes, or a target temperature selected in the range from 30°C up to 70°C for a duration selected in the range from 1 minute up to 5 minutes, or a target temperature selected in the range from 30°C up to 55°C for a duration selected in the range from 1 minute up to 5 minutes.

While the device according to the invention may be used for chemical reaction vessels of any custom dimensions, embodiments disclosed herein include all embodiments of devices with dimensions suitable to be used to SPE cartridges as known to the skilled person. In embodiments wherein the device comprises a metal sleeve, the metal sleeve may facilitate the adaptation of the device to chemical reaction vessels with different maximum dimension of cross section, e.g., different tubular chemical reaction vessels with different diameters. Example dimensions for the metal sleeve are a length from 10 up to 100 mm, e.g., 25 mm and/or an inner diameter from 2 mm up to 30 mm, e.g., 11 mm and/or an outer diameter from 3 mm up to 35 mm, e.g., 15.9 or 16 mm. In embodiments, the length and/or resistance value and/or maximum power of the resistive conductor is adapted to the dimensions and/or requirements of the chemical reaction vessel chosen for use with the device and/or in function of the predetermined temperature requirement.

In embodiments, the maximum power (also known as power rating) of the resistive conductor is 150 W, 140 W, 130 W, 120 W, 110 W, 100 W, 90 W, 80 W, 70 W, 60 W, 50 W, 40 W, 30 W, 20 W, 10 W, or 5 W.

In embodiments, the insulator polymer of the heating means or heat pad comprises a silicone or silicone rubber. Advantages of such material include that it provides a device and a heat pad that may be rugged, moisture-resistant and/or chemical-resistant. Moreover, such heat pads may be easily can be bonded to other parts of the device for effective heat transfer. Silicone rubber is advantageously capable of withstanding extremely high temperatures without deterioration. Moreover, silicone rubber insulation provides an adequate waterproof connection. In embodiments, the insulator polymer comprising silicone and/or devices comprising an insulator polymer comprising silicone are suitable for temperatures of more than 50°C, preferably more than 100°C, more preferably more than 200°C, most preferably more than 250°. In preferred embodiments, the insulator polymer comprising silicone and/or devices comprising an insulator polymer comprising silicone are suitable for temperatures from 50°C, or from 100°C, or from 200°C, up to 300°C. In preferred embodiments, the melting temperature of said insulator polymer is higher than 150°C, preferably higher than 200°C.

In embodiments, the insulator polymer of the heating means comprises a polyimide. Advantages of such material is that it is a thin and a lightweight transparent material, providing a thin and lightweight device. In embodiments, the insulator polymer comprising a polyimide and/or devices comprising an insulator polymer comprising a polyimide are suitable for temperatures
- of more than 50°C, preferably more than 100°C, more preferably more than 150°C, most preferably 200°C and/or
- of less than 400°C, preferably less than 350°C, more preferably less than 300°C, and/or
- within a range of 50°C to 400°C, preferably within a range of 50°C to 350°C, more preferably of 50°C to 300°C.

In embodiments, the heating means comprises a wire wound element embedded or, equivalently, encased in the polymer insulator. The advantages of such a wire-wound element include physical strength and flexibility, wherein repeated heater flexing has no harmful effects on its performance. Another advantage relates to the ability to conform easily to curved surfaces, including small radius bends, allowing for a slim device and/or a device with a low diameter. Another advantage is that the heater may be flexed repeatedly during removal and installation, with no internal damage occurring, leading to ease of manufacturing.

In embodiments, the resistive conductor of the heating means has an insulation resistance of more than 100 KΩ, preferably more than 1 MΩ, preferably at least 10 MΩ.

In embodiments, the heating means comprises an etched foil element embedded or, equivalently, encased in the polymer insulator. The advantages of such an edged foil element include good circuit pattern repeatability and superior heat transfer, which may result from greater coverage of the element. Other advantages relate to delivery of increased heat and/or increased watt density and/or increased heater life. Another advantage relates to the ability to create complex heat distribution patterns and/or multiple zoning patterns.

In embodiments, the heating means is a flexible sheet,
- wherein preferably said flexible sheet has a rectangular shape; and/or
- wherein preferably a thickness of said flexible sheet is from 0.5 mm up to 1.5 mm, more preferably from 0.5 mm up to 1.0 mm.

In embodiment, the heating means is a flexible sheet, wherein said flexible sheet has a rectangular shape, wherein said rectangular shape has a width from 20 mm up to 200 mm and a length of 20 mm up to 200 mm, or a width from 20 mm up to 150 mm and a length of 20 mm up to 150 mm, or a width from 20 mm up to 100 mm and a length of 20 mm up to 100 mm, preferably wherein a thickness of said flexible sheet is from 0.5 mm up to 1.5 mm, more preferably from 0.5 mm up to 1.0 mm.

In embodiments, the heating means is a flexible sheet, wherein said flexible sheet has a rectangular shape, wherein:
- said rectangular shape has a width from 20 mm up to 30 mm and a length of 45 mm up to 55 mm, preferably a width of 25 mm and a length of 50 mm, preferably wherein the maximum power of the resistive conductor is 5 W; or
- said rectangular shape has a width from 45 mm up to 55 mm and a length of 45 mm up to 55 mm, preferably a width of 50 mm and a length of 50 mm, preferably wherein the maximum power of the resistive conductor is 10 W; or
- said rectangular shape has a width from 45 mm up to 55 mm and a length of 70 mm up to 80 mm, preferably a width of 50 mm and a length of 75 mm, preferably wherein the maximum power of the resistive conductor is 15 W; or
- said rectangular shape has a width from 45 mm up to 55 mm and a length of 95 mm up to 105 mm, preferably a width of 50 mm and a length of 100 mm, preferably wherein the maximum power of the resistive conductor is 20 W; or
- said rectangular shape has a width from 45 mm up to 55 mm and a length of 145 mm up to 155 mm, preferably a width of 50 mm and a length of 150 mm, preferably wherein the maximum power of the resistive conductor is 30 W; or
- said rectangular shape has a width from 70 mm up to 80 mm and a length of 95 mm up to 1052 mm, preferably a width of 75 mm and a length of 100 mm, preferably wherein the maximum power of the resistive conductor is 30 W; or
- said rectangular shape has a width from 95 mm up to 105 mm and a length of 95 mm up to 105 mm, preferably a width of 100 mm and a length of 100 mm, preferably wherein the maximum power of the resistive conductor is 40 W; or
- said rectangular shape has a width from 95 mm up to 105 mm and a length of 145 mm up to 155 mm, preferably a width of 100 mm and a length of 150 mm, preferably wherein the maximum power of the resistive conductor is 60 W; or
- said rectangular shape has a width from 70 mm up to 80 mm and a length of 195 mm up to 205 mm, preferably a width of 75 mm and a length of 200 mm, preferably wherein the maximum power of the resistive conductor is 60 W; or
- said rectangular shape has a width from 145 mm up to 155 mm and a length of 195 mm up to 205 mm, preferably a width of 150 mm and a length of 200 mm, preferably wherein the maximum power of the resistive conductor is 120 W;
preferably wherein a thickness of said flexible sheet is from 0.5 mm up to 1.5 mm.

In embodiments, said flexible sheet comprises a reinforcement layer covering one side of said flexible sheet, preferably wherein said reinforcement layer consists of glass and/or fiber glass. This has the advantage of providing enhanced structural integrity.

In embodiments, the device according to the invention comprises a metal sleeve placed between the chemical reaction vessel and the heating means and surrounding the chemical reaction vessel, the metal preferably being copper. In embodiments, the metal sleeve may relate to a replaceable or exchangeable metal sleeve taken from a plurality of metal sleeves, wherein the plurality is characterized by at least two different thicknesses and/or at least two different inner or outer diameters. Thereby, the presence of a metal sleeve facilitates the adaptation of the device to chemical reaction vessels with different maximum dimension of cross section, e.g., different tubular chemical reaction vessels with different diameters.

In embodiments, the device according to the invention comprises a metal sleeve placed between the chemical reaction vessel and the heating means and surrounding the chemical reaction vessel, the metal being preferably copper, wherein the mass of the metal sleeve is from 10 g up to 550 g, preferably from 20 up to 550 g.

In embodiments, the device according to the invention comprises a metal sleeve placed between the chemical reaction vessel and the heating means and surrounding the chemical reaction vessel, the metal being preferably copper, wherein the ratio between the maximum power of the resistive conductor and the mass of said metal sleeve is 0.5 W/g, 0.45 W/g, 0.4 W/g, 0.35 W/g, 0.3 W/g, 0.25 W/g, 0.2 W/g, 0.15 W/g, or 0.1 W/g.

In embodiments. the device according to the invention comprises a metal sleeve placed between the chemical reaction vessel and the heating means and surrounding the chemical reaction vessel, the metal being preferably copper, wherein the mass of the metal sleeve is from 10 g up to 550 g, wherein the ratio between the maximum power of the resistive conductor and the mass of said metal sleeve is 0.5 W/g, 0.45 W/g, 0.4 W/g, 0.35 W/g, 0.3 W/g, 0.25 W/g, 0.2 W/g, 0.15 W/g, or 0.1 W/g.

In embodiments, the device according to the invention furthermore comprises a cover. The cover may conveniently keep the device together. The cover may comprise one or more holes, e.g. for the power supply to the heating means, which may provide for more robustness for the electrical connection of the heating means, including reduced risk of tear.

In embodiments, the heating means comprises an adhesive layer for attaching said heating means to further portions of said device according to the invention, preferably for attaching said heating means to said metal sleeve or a cover surrounding said heating means. This has the advantage of easier mounting of the device and/or improved structural integrity of the device and/or improved thermal conductivity for heat propagating from the heating means to the metal sleeve.

In embodiments, the device according to the invention comprises a control unit wherein said control unit is configured to maintain said predetermined temperature requirement relating to a temperature of said mixture being in a predetermined subrange comprised in a range from 30°C up to 250°C, from 30°C up to 250°C, from 30°C up to 240°C, from 30°C up to 230°C, from 30°C up to 220°C, from 30°C up to 210°C, from 30°C up to 200°C, from 30°C up to 190°C, from 30°C up to 180°C, from 30°C up to 170°C, from 30°C up to 160°C, from 30°C up to 150°C, from 30°C up to 140°C, from 30°C up to 130°C, from 30°C up to 120°C, from 30°C up to 110°C, from 30°C up to 100°C, from 30°C up to 90°C, from 30°C up to 80°C, from 30°C up to 70°C, from 30°C up to 60°C, from 30°C up to 50°C, by controlling the power supplied to said heating means. The control unit has the advantage of controlling the current of electrical energy from the power source to the heating means. In embodiments, the control unit comprises a relay, providing a simple and reliable means of controlling heat supply. In embodiments, the control unit comprises, preferably consists of, a programmable logical controller that is connected to the heating means via insulated lead, preferably PTFE insulated lead. In embodiments, the device further comprises a temperature sensor, and said controlling is based on measurement by said temperature sensor. This provides the advantage of increased control of the heat provided to the chemical reaction vessel. This may relate to more accurately and/or more rapidly complying with the predetermined temperature requirement, e.g., by preventing overshoot and/or reaching a stable temperature quickly and after a short equilibration time. In embodiments, the temperature sensor, which may comprise one or more sensor components, is positioned on a portion of the heating means and/or placed between the heating means and the metal sleeve (if present) and/or between the heating means and the chemical reaction vessel and/or on a further portion of the chemical reaction vessel such as portions near the inlet or outlet.

In embodiments, said predetermined temperature requirement is met by means of a control unit being a programmable logical controller (PLC). Preferably, temperature is measured by means of a temperature sensor comprising a thermocouple board of the PLC. Preferably, the control unit is configured such that temperature is controlled according to a PID control. This relates to specific parameters configured to avoid temperature overshoot.

In embodiments, the device according to the invention is a tubular sleeve and said opening is a lumen surrounded by said device.

In embodiments, the chemical reaction vessel, comprised in a system according to the invention, further comprises an outlet (32). In embodiments, the chemical reaction vessel is an SPE cartridge.

In embodiments, the chemical reaction vessel, comprised in a system according to the invention, has a height from 5 mm up to 500 mm, or from 5 mm up to 450 mm, or from 5 mm up to 400 mm, or from 5 mm up to 350 mm, or from 5 mm up to 300 mm, or from 5 mm up to 250 mm, or from 5 mm up to 200 mm or from 5 mm up to 150 mm, or from 5 mm up to 100 mm.

In embodiments, the chemical reaction vessel, comprised in a system according to the invention, has a diameter from 1 mm up to 200 mm, or from 1 mm up to 190 mm, or from 1 mm up to 180 mm, or from 1 mm up to 170 mm, or from 1 mm up to 160 mm, or from 1 mm up to 150 mm, or from 1 mm up to 140 mm, or from 1 mm up to 130 mm, or from 1 mm up to 120 mm, or from 1 mm up to 110 mm, or from 1 mm up to 100 mm, or from 1 mm up to 90 mm, or from 1 mm up to 80 mm, or from 1 mm up to 70 mm, or from 1 mm up to 60 mm, or from 1 mm up to 50 mm.

In embodiments, the chemical reaction vessel comprised in a system according to the invention, has a diameter from 11 mm up to 12 mm and a height from 10.5 mm up to 11.5 mm. Preferably, said diameter is 11.5 mm and said height is 11mm.

In embodiments, the chemical reaction vessel comprised in a system according to the invention, has a diameter from 11 mm up to 12 mm and a height from 19.5 mm up to 20.5 mm. Preferably, said diameter is 11.5 mm and said height is 20mm.

In embodiments, a volume enclosed by the chemical reaction vessel, comprised in a system according to the invention, is from 100 µm to 1mL, preferably from 200 µL up to 800 µL, more preferably from 300 µL up to 700 µL.

In embodiments, the housing of the chemical reaction vessel, comprised in a system according to the invention, comprises a metal, and/or a metal alloy and/or glass and/or fiber glass and/or an organic polymer. In embodiments, said housing consists of one or more organic polymers.

In embodiments, said solid phase, comprised in the chemical reaction vessel of a system according to the invention, is a silica, preferably said solid phase comprises one or more of Sep-Pak tC18, Step-Pak C18, Oasis HLB, Oasis MCX, Oasis MAX and Sephadex LH-20, preferably said solid phase is Sep-Pak tC18.

In embodiments, the mass of said solid phase, comprised in the chemical reaction vessel of a system according to the invention is from 1 mg up to 1 g.

### Definitions

Each embodiment as identified herein may be combined together unless otherwise indicated. All patent and literature references cited in the present specification are hereby incorporated by reference in their entirety.

In this document and in its claims, the verb "to comprise" and its conjugations is used in its non-limiting sense to mean that items following the word are included, but items not specifically mentioned are not excluded. In addition, the verb "to consist" may be replaced by "to consist essentially of" meaning that a method as defined herein may comprise additional steps or components) than the ones specifically identified, said additional component(s) not altering the unique characteristic of the invention. In addition, reference to an element by the indefinite article "a" or "an" does not exclude the possibility that more than one of the element is present, unless the context clearly requires that there be one and only one of the elements. The indefinite article "a" or "an" thus usually means "at least one".

In the context of this invention, a decrease or increase of a parameterto be assessed means a change of at least 5% of the value corresponding to that parameter. More preferably, a decrease or increase of the value means a change of at least 10%, even more preferably at least 20%, at least 30%, at least 40%, at least 50%, at least 70%, at least 90%, or 100%. In this latter case, it can be the case that there is no longer a detectable value associated with the parameter.

Wherever a list of numbers is provided like "at least X, Y, or Z" and "less than X%, Y%, Z%", such statements should be interpreted as "at least X, or at least Y, or at least Z" and "less than X%, or less than Y%, or less than Z%", respectively.

The use of a substance as a medicament as described in this document can also be interpreted as the use of said substance in the manufacture of a medicament. Similarly, whenever a substance is used for treatment or as a medicament, it can also be used for the manufacture of a medicament for treatment.

The word "about" or "approximately" when used in association with a numerical value (e.g. about 10) preferably means that the value may be the given value (of 10) more or less 0.1% of the value.

The proposition "between" when used in association with integers refers to a range including the boundary values mentioned. For example, if n is a value between 1 and 3, n may be 1, 2 or 3. In other words, "between X and Y" is a synonym of "from X up to Y".

In the context of this invention, "represented by structure X", "of structure X" and "with structure X" are used interchangeably.

The term "affinity", as used herein, refers to the degree to which a polypeptide, in particular an immunoglobulin, such as an antibody, or an immunoglobulin fragment, such as a VHH, binds to an antigen so as to shift the equilibrium of antigen and polypeptide toward the presence of a complex formed by their binding. Thus, for example, where an antigen and antibody (e.g., antibody fragment) are combined in relatively equal concentration, an antibody (e.g., antibody fragment) of high affinity will bind to the available antigen so as to shift the equilibrium toward a high concentration of the resulting complex. The dissociation constant (KD) is commonly used to describe the affinity between the protein binding domain and the antigenic target. Typically, the dissociation constant is lower than 10⁻⁵ M. In some embodiments, the dissociation constant between an antigen and a biological moiety used in a method for attaching a biological moiety according to the invention is lower than 10⁻⁶ M, more preferably, lower than 10⁻⁷ M, most preferably, lower than 10⁻⁸ M, such as lower than 10⁻⁹ M.

The terms "specifically bind" and "specific binding", as used herein, generally refers to the ability of a polypeptide, in particular an immunoglobulin, such as an antibody, or an immunoglobulin fragment, such as a VHH or fragments thereof, which may be used in as biological moiety in a method for attaching a biological moiety according to the invention, to preferentially bind to a particular antigen that is present in a homogeneous mixture of different antigens. In certain embodiments, a specific binding interaction will discriminate between desirable and undesirable antigens in a sample, in some embodiments more than about 10 to 100-fold or more (e.g., more than about 1000- or 10,000-fold).

Accordingly, an amino acid sequence, in particular an antibody fragment, such as a VHH or fragments thereof, as disclosed herein as biological moiety used in a method for attaching a biological moiety according to the invention, is said to "specifically bind to" a particular target when that amino acid sequence has affinity for, specificity for and/or is specifically directed against that target (or for at least one part or fragment thereof).

The "specificity" of an amino acid sequence, in particular an antibody fragment, such as a VHH, or fragments thereof, which may be used as a biological moiety in a method for attaching a biological moiety according to the invention, can be determined based on affinity and/or avidity. The "affinity" of an amino acid sequence, comprised in a biological moiety used in method for attaching a biological moiety according to the invention, is represented by the equilibrium constant for the dissociation of the amino acid sequence, which may be a biological moiety used in a method for attaching a biological moiety according to the invention, and the target protein of interest to which it binds. The lower the KD value, the stronger the binding strength between the amino acid sequence, which may be comprised in a biological moiety used in a method for attaching a biological moiety according to the invention, and the target protein of interest to which it binds. Alternatively, the affinity can also be expressed in terms of the affinity constant (KA), which corresponds to 1/KD. The binding affinity of an amino acid sequence, which may be comprised in a biological moiety used in a method for attaching a biological moiety according to the invention, can be determined in a manner known to the skilled person, depending on the specific target protein of interest. The "avidity" of an amino acid sequence, which may be comprised in a biological moiety used in a method for attaching a biological moiety according to the invention, is the measure of the strength of binding between said amino acid sequence and the pertinent target protein of interest. Avidity is related to both the affinity between a binding site on the target protein of interest and a binding site on the amino acid sequence and the number of pertinent binding sites present on the amino acid sequence. In some embodiments, the amino acid sequences comprised in a biological moiety used in a method for attaching a biological moiety according to the invention will bind to a target protein of interest with a dissociation constant (KD) of less than about 1 micromolar (1 µM), and preferably less than about 1 nanomolar (1 nM) [i.e., with an association constant (KA) of about 1,000,000 per molar (10⁶ M⁻¹, 1E6 /M) or more and preferably about 1,000,000,000 per molar (10⁹ M⁻¹, 1E9 /M) or more]. A KD value greater than about 1 millimolar is generally considered to indicate non-binding or non-specific binding. It is generally known in the art that the KD can also be expressed as the ratio of the dissociation rate constant of a complex, denoted as k0ff (expressed in seconds⁻¹ or s⁻¹), to the rate constant of its association, denoted k0n (expressed in molar⁻¹ seconds⁻¹ or M⁻¹ s⁻¹). In some embodiments, an amino acid sequence, comprised in a biological moiety used in a method for attaching a biological moiety according to the invention, will bind to the target protein of interest with a koff ranging between 0.1 and 0.0001 s⁻¹ and/or a k0n ranging between 1,000 and 1,000,000 M⁻¹ s⁻¹. Binding affinities, k0ff and k0n rates may be determined by means or methods known to the person skilled in the art, for example ELISA methods, isothermal titration calorimetry, surface plasmon resonance, fluorescence-activated cell sorting analysis, and the more.

In respect of the amino acid sequences, in particular antibody fragments, such as a VHH or fragments thereof, which may be used as a biological moiety in a method for attaching a biological moiety according to the invention, the terms "binding region", "binding site" or "interaction site" present on the amino acid sequences, which may be comprised in a biological moiety used in a method for attaching a biological moiety according to the invention, shall herein have the meaning of a particular site, part, domain or stretch of amino acid residues present in the amino acid sequence that is responsible for binding to a target molecule. In some embodiments, such binding region essentially consists of specific amino acid residues from the amino acid sequence which are in contact with the target molecule.

In cases where all of the two or more binding sites of amino acid sequence, in particular an antibody fragment, such as a VHH or fragments thereof, which may be a biological moiety used in a method for attaching a biological moiety according to the invention, are directed against or specifically bind to the same site, determinant, part, domain or stretch of amino acid residues of the target of interest, the amino acid sequence is said to be "bivalent" (in the case of two binding sites on the amino acid sequence) or multivalent (in the case of more than two binding sites on the amino acid sequence), such as for example trivalent.

As used herein, the term "monovalent" when referring to an antibody fragment, such as a VHH or fragment thereof, denotes an antibody fragment in monomeric form. A monovalent antibody fragment contains only one binding site. In this context, the binding site of an antibody fragment, such as a VHH or fragments thereof, encompasses the one or more "complementarity determining regions" or "CDRs" of an antibody fragment that are directed against or specifically bind to a particular site, determinant, part, domain or stretch of amino acid residues of a target of interest.

As used herein, the term 'untagged' when referring to an antibody fragment, such as a VHH or fragments thereof, denotes an antibody fragment that contains no extraneous polypeptide sequences. Exemplary extraneous polypeptide sequences include carboxy-terminal polypeptide tags, e.g., a His-tag, a cysteine-containing tag (e.g., a GGC-tag), and/or a Myc-tag.

The term "bi-specific" when referring to an amino acid sequence, in particular an antibody fragment, such as a VHH, which may be a biological moiety used in a method for attaching a biological moiety according to the invention, implies that either a) two or more of the binding sites of an amino acid sequence are directed against or specifically bind to the same target of interest but not to the same (i.e. to a different) site, determinant, part, domain or stretch of amino acid residues of that target, the amino acid sequence is said to be "bi-specific" (in the case of two binding sites on the amino acid sequence) or multispecific (in the case of more than two binding sites on the amino acid sequence) or b) two or more binding sites of an amino acid sequence are directed against or specifically bind to different target molecules of interest. The term "multispecific" is used in the case that more than two binding sites are present on the amino acid sequence.

In some embodiments, a "bispecific" amino acid sequence or antibody fragment, such as a "bispecific" VHH or a "multi-specific" amino acid sequence or antibody fragment, such as a "multispecific" VHH, which may be a biological moiety used in a method for attaching a biological moiety according to the invention, shall have the meaning of an amino acid sequence, in particular an antibody fragment, such as a VHH, which may be a biological moiety used in a method for attaching a biological moiety according to the invention, comprising respectively two or at least two binding sites, wherein these two or more binding sites have a different binding specificity. In some embodiments, an amino acid sequence, in particular an antibody fragment, such as a VHH, which may be a biological moiety used in a method for attaching a biological moiety according to the invention, is considered "bispecific" or "multispecific" if respectively two or more than two different binding regions exist in the same, monomeric, amino acid sequence.

The "half-life" of an amino acid sequence, in particular an antibody fragment, such as a VHH or fragments thereof, which may be a biological moiety used in a method for attaching a biological moiety according to the invention, can generally be defined as the time that is needed for the in vivo serum concentration of the amino acid sequence to be reduced by 50%. The in vivo half-life of an amino acid sequence can be determined in any manner known to the person skilled in the art, such as by pharmacokinetic analysis. As will be clear to the skilled person, the half-life can be expressed using parameters such as the tl/2-alpha, tl/2-beta and the area under the curve (AUC). An increased half-life in vivo is generally characterized by an increase in one or more and preferably in all three of the parameters tl/2-alpha, tl/2-beta and the area under the curve (AUC).

The term "lifetime extended" when referring to an antibody fragment, such as a VHH or fragments thereof, which may be a biological moiety used in a method for attaching a biological moiety according to the invention, is used to denote that the antibody fragment has been modified to extend the half-life of the antibody fragment. Strategies for extending the half-life of antibodies and antibody fragments are well-known in the art and include for example, but without limitation, linkage (chemically or otherwise) to one or more groups or moieties that extend the half-life, such as polyethylene glycol (PEG), bovine serum albumin (BSA), human serum albumin (HSA), antibody Fc fragments, or antigen-binding antibody fragments targeting serum proteins such as serum albumin.

A range from a given width centered around a given value is the closed interval of values from said given value minus half said width up to said given value plus half said given width. For example, if a temperature is selected in the range from 10°C centered around 35°C, it is meant that said temperature is from 35°C (35°C - 10°C / 2) up to 40°C (35°C + 10°C / 2).

### Legend to the figures

**Figure 1** - Overview of the synthesis of [¹³¹I-SGMIB.
**Figure 2** - Schematic description of the tC18 Sep-Pak cartridge deprotection procedure.
**Figure 3** - Evolution of the yield of the fully deprotected product as a function of the heating temperature, wherein said heating is for 20 minutes.
**Figure 4** - Evolution of the yield of the fully deprotected product as a function of the heating temperature, wherein said heating is for 5 minutes.
**Figure 5** - Example of the cassette used for the automation of heated deprotection of Boc₂-protected [*I]I-SGMIB.
**Figure 6** - Different views of an example embodiment of a device and system according to the invention. Figure 1a shows a first cut away view. Figure 1b shows a perspective view. Figure 1c shows a second cut away view.
**Figure 7** - Example embodiments of a heating element comprised in a system according to the invention. Figure 2a shows an example of a heat pad comprising a wire wound heating element. Figure 2b shows an example of a heat pad comprising an etched foil heating element.
**Figure 8** - The simulated effect of power the heating means comprised in an example embodiment of a device according to the invention (SolidWorks). Figure 3a shows a thermal distribution of said device. Figure 3b show the temperature of said device as a function of time of powering said heating means.
**Figure 9** - The simulated effect of power the heating means comprised in an example embodiment of a system according to the invention (SolidWorks). Figure 4a shows a thermal distribution of said system. Figure 4b show the temperature of said system as a function of time of powering said heating means.

### References

[1] MOSDZIANOWSKI, C., et al. Epimerization study on [18F] FDG produced by an alkaline hydrolysis on solid support under stringent conditions. Applied radiation and isotopes, 2002, 56.6: 871-875.
[2] US8476063B2
[3] US20020183660A1
[4] US9408257B2
[5] KR101320762B1

### Examples

The following examples are offered for illustrative purposes only, and are not intended to limit the scope of the present invention in any way.

### Example 1: Manual reactions

### Manual process description

The set of experiments described herein aimed at reproducing the preliminary results obtained for the "cold" synthesis of the SGMIB linker using a heated cartridge during the deprotection step (= cold experiments conducted by ORA). Following this first proof-of-concept, a series of manual "hot" syntheses using a heated cartridge were conducted (= radioactive experiments conducted by Camel-IDS), which are described in detail below.

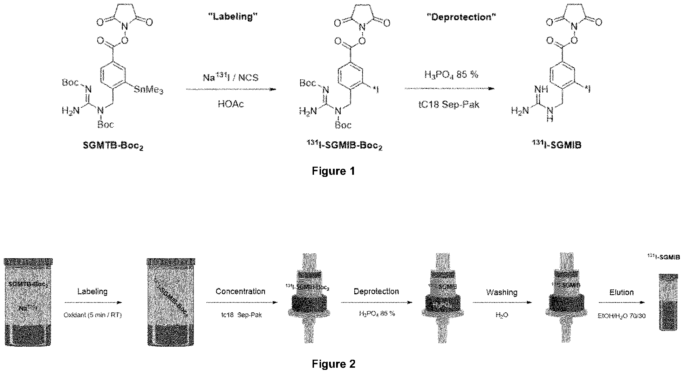

The examples detailed below describe the performance of the novel method on the deprotection of Boc2-N-succinimidyl-4-guanidinomethyl-3-[(*)I]iodobenzoate ([*I]SGMIB-BOC₂), a radiohalogenation agent used for radiolabeling of targeting compounds. [*I]SGMIB is obtained via the synthesis procedure described in Figure 1. A detailed description of tC18 Sep-Pak cartridge deprotection procedure us depicted in Figure 2.

With the presented set of experiments, the potency of deprotection via a method according to the invention a heated cartridge is evaluated at different reaction temperatures and will be evaluated in function of reaction times and the type of acid (and its concentration) used.

In process measurement of deprotection potency and quality of the radioactive compounds are assessed by gradient RP- HPLC analyses. Identical chromatographic conditions are used to assess the fully automated procedure.

### Materials and methods

All reagents (*N*-chlorosuccinimide (NCS), tin-precursor (SGMTB-Boc₂), sodium iodide (Na¹²⁷I), phosphoric acid 85 % (H₃PO₄), acetic acid (HOAc)) and solvents (acetonitrile (ACN), ethanol (EtOH)) were obtained commercially from Merk-Sigma and used without further purification. For deprotection, Sep-Pak C18 Plus Short/Light Cartridge were obtained commercially from Waters.

Na[¹³¹I]I was obtained commercially from GE Healthcare in 0.05 M NaOH solution with a volumic activity of 800 µCi/µL (29.6 MBq/µL). UV/Radio-HPLC analyses were performed on a Shimadzu LC-20AT Liquid chromatography system equipped with a C-18 column (X-Select, CSH, C18, 3.5 µ, 100 x 4.6 mm) with the flow rate set at 1.50 mL/min with the following gradient: t=0: 90 % A, 10 % B; t=15 min: 100% B; with A = H₂O with 0.05% TFA and B = ACN with 0.05% TFA.

### General experimental protocol

### a) Na¹³¹ preparation

To a 10 mL vial:
1. Adding 20 µL of H₂O (WFI)
2. Adding *desired* amount of cold iodine (Na¹²⁷I)
3. Adding 5 µL of 10 x diluted PBS solution
4. Adding *desired* activity of radioactive iodine (Na¹³¹I) **→** evaporation at 45 °C until complete drying

### b) Labeling step

To the dry Na¹³¹I (10 mL vial):
1. Adding *desired* oxidizing reagent solution (NCS)
2. Adding *desired* tin precursor solution (SGMTB-Boc₂) **→** Labeling time: 5 min / 23 °C
3. QC HPLC: 10 µL of labeled solution in 90 µL of H₂O + 0.05 % TFA (vᵢₙⱼ: 100 µL)

### c) Concentration and deprotection steps (SGMIB-Boc₂)

To the Labeled solution (10 mL vial):
1. Dilution with *desired* volume of H₂O (WFI)
2. Loading diluted labeled solution on *desired* tC18 cartridge
3. Rinsing tC18 cartridge with *desired* volume of H₂O (WFI)
4. Drying tC18 cartridge with air
5. Adding *desired* volume of phosphoric acid 85 % (H₃PO₄) in tC18 cartridge
6. Adding tC18 cartridge to a water bath after being closed with two stoppers **→** Deprotection time: 20 min / *desired* T°C

### d) Elution final product step (SGMIB)

To the tC18 cartridge (Sep-Pak):
7. Washing with *desired* volume of H₂O (WFI)
8. Drying with air
9. Eluting final product (¹³¹I-SGMIB) with *desired* volume of EtOH/H₂O (70/30) + 1 % HOAc
10. QC HPLC: 10 µL of eluted solution in 90 µL of H₂O + 0.05 % TFA (vᵢₙⱼ: 100 µL)

### Experimental conditions (manual) + Results tables

As mentioned above, with the presented set of experiments, the potency of deprotection using a heated cartridge is evaluated at different reaction temperatures, ranging from 23°C up to 75°C. The corresponding results (Deprotection yields) can be found in the table depicted below. The relevant RP-HPLC chromatograms are described part 3.

The first results indicate that for a deprotection time of 20 min within a temperature range of 23°C - 75°C, deprotection reaction yield measures > 75%. Within a 32°C - 75°C range, the deprotection yield increases > 92%, while it measures >99% between 40°C - 75°C.

For a deprotection time of 5 min and a temperature range of 23°C - 55°C, deprotection reaction yield measures > 30%. Within a 40°C - 75°C range, the yield increases > 73%, while it measures > 99% between 55°C - 75°C.

Importantly, efficient heated cartridge deprotection was confirmed for [*I]SGMIB that was radioiodinated in a reaction mixture consisting of either EtOH/HOAc or ACN/HOAc. These experiments indicated that the observed deprotection efficiency was independent of the constitution of the radioiodination reaction mixture (which takes place before deprotection).

Table 1 describes the set-up of the experiments, whereas Table 2 lists the results obtained for each of these experiments.

In addition, RP-HPLC results indicate that when the deprotection reaction takes place at 75°C, an impurity to [*I]SGMIB is observed, ranging from 3 - 12%. Deprotection reactions at 40°C do not give rise to this impurity (< 1%).

**Table 1 (part 1): Set-up of the manual experiments**

| Step | Exp | 1 | 2 | 3 | 4 | 5 | 6 | 7 | 8 |
|---|---|---|---|---|---|---|---|---|---|
| | Date | 2020-03-16 | 2020-03-18 | 2020-03-19 | 2020-0416 | 2020-04-17 | 2020-04-16 | 2020-04-17 | 2020-04-17 |
| Drying step | Radio. isotope | ¹³¹I | | | ¹³¹I | | | ¹³¹I | |
| | Na¹²⁷I (eq) | 100 mCi | 1 Ci | 1 Ci | 5 Ci | | | 5 Ci | |
| | Drying time | 10 min | | | 10 min | | | 10 min | |
| | Drying T°C | 45 °C | | | 45 °C | | | 45 °C | |

| Lab. step | Oxidant (NCS) | 1.8 mg in 600 µL EtOH/HOAc (99/1) | | | 3.75 mg in 1.25 mL EtOH/HOAc (87.5/12.5) | | | 3.75 mg in 1.25 mL ACN/HOAc (87.5/12.5) | |
|---|---|---|---|---|---|---|---|---|---|
| | Tin precursor | 0.48 mg in 600 µL EtOH/HOAc (99/1) | | | 1.5 mg in 1.25 mL EtOH/HOAc (87.5/12.5) | | | 1.5 mg in 1.25 mL ACN/HOAc (87.5/12.5) | |
| | End volume | 1.2 mL | | | 2.5 mL | | | 2.5 mL | |
| | Lab. time | 20 min | | | 5 min | | | 5 min | |
| | Lab. T°C | 23 °C | | | 23 °C | | | 23 °C | |
| Conc. + dep. steps | H₂O (dilution) | 4 mL | | | 6 mL | 8 mL | 6 mL | 8 mL | |
| | tC18 Sep-Pak type | Short | Light | | Short | | | Short | |
| | H₂O (rinsing) | 5 mL | | | 5 mL | | | 5 mL | |
| | H3PO4 85 % | 2 mL | | | 2 mL | | | 2 mL | |
| | Dep. time | 20 min | | | 20 min | | | 20 min | |
| | Dep. T°C | 75 °C | | | 40 °C | 32 °C | 23 °C | 40 °C | 32 °C |
| Elution step | H₂O (rinsing) | 8 mL | 10 mL | 10 mL | 15 mL | | | 15 mL | |
| | Eluent | EtOH/H₂O (70/30) + 1 % HOAc | | | EtOH/H₂O (70/30) + 1 % HOAc | | | EtOH/H₂O (70/30) + 1 % HOAc | |

| | Elution mode | Straight | Straight | Reverse | Straight | | | Straight | |
|---|---|---|---|---|---|---|---|---|---|
| | Eluent volume | 3.5 mL | 1.5 mL | 1.0 mL | 2.0 mL | | | 2.0 mL | |

**Table 1 (part 2): Set-up of the manual experiments**

| Step | Exp | 9 | 10 | 11 | 12 | 13 | 14 |
|---|---|---|---|---|---|---|---|
| | Date | 2020-04-22 | 2020-04-21 | 2020-04-21 | 2020-04-21 | 2020-04-22 | 2020-04-22 |
| Drying step | Radio. isotope | ¹³¹I | | | | | ¹³¹I |
| | Na¹²⁷I (eq) | 5 Ci | | | | | 5 Ci |
| | Drying time | 10 min | | | | | 10 min |
| | Drying T°C | 45 °C | | | | | 45 °C |
| Lab. step | Oxidant (NCS) | 3.75 mg in 1.25 mL EtOH/HOAc (87.5/12.5) | | | | | 3.75 mg in 1.25 mL ACN/HOAc (87.5/12.5) |
| | Tin precursor | 1.5 mg in 1.25 mL EtOH/HOAc (87.5/12.5) | | | | | 1.5 mg in 1.25 mL ACN/HOAc (87.5/12.5) |
| | End volume | 2.5 mL | | | | | 2.5 mL |
| | Lab. time | 5 min | | | | | 5 min |
| | Lab. T°C | 23 °C | | | | | 23 °C |
| Conc. + dep. steps | H₂O (dilution) | 8 mL | | | | | 8 mL |
| | tC18 Sep-Pak type | Short | | | | | Short |
| | H₂O (rinsing) | 5 mL | | | | | 5 mL |
| | H3PO4 85 % | 2 mL | | | | | 2 mL |
| | Dep. time | 5 min | | | | | 5 min |
| | Dep. T°C | 23 °C | 55 °C | 45 °C | 50 °C | 55 °C | |
| Elution step | H₂O (rinsing) | 15 mL | | | | | 15 mL |
| | Eluent | EtOH/H₂O (70/30) + 1 % HOAc | | | | | EtOH/H₂O (70/30) + 1 % HOAc |
| | Elution mode | Straight | | | | | Straight |
| | Eluent volume | 2.0 mL | | | | | 2.0 mL |

**Table 2 (part 1): Results of the manual experiments**

| Exp | 1 | 2 | 3 | 4 | 5 | 6 | 7 | 8 |
|---|---|---|---|---|---|---|---|---|
| Starting Activity (MBq) | 80.6 | 83.2 | 79.6 | 48.1 | 44.2 | 49.0 | 43.6 | 30.1 |
| Collected activity (MBq) | 70 | 71 | 67.4 | 41.8 | 38.9 | 42.2 | 38.7 | 27.5 |
| Deprotection yield (%) | > 99 | > 99 | > 99 | > 99 | 92 | 75 | > 99 | 92 |
| SGMIB yield (purity (%)) | 87 | 96 | 95 | 99 | 92 | 75 | 99 | 92 |
| SGMIB (MBq) | 60.9 | 68.2 | 64.0 | 41.4 | 35.8 | 31.7 | 38.3 | 25.3 |
| Overall yield (%) | 76 | 82 | 80 | 86 | 81 | 65 | 88 | 84 |
| Collected volume (mL) | 3.4 | 1.4 | 0.8 | 2.2 | 2.1 | 2.1 | 2.1 | 2.2 |

**Table 2 (part 2): Results of the manual experiments**

| Exp | 9 | 10 | 11 | 12 | 13 | 14 |
|---|---|---|---|---|---|---|
| Starting Activity (MBq) | 24.0 | 29.2 | 29.1 | 29.2 | 23.6 | 23.9 |
| Collected activity (MBq) | 21.2 | 26.9 | 26.4 | 26.7 | 21.7 | 21.5 |
| Deprotection yield (%) | 30 | 73 | 91 | 96 | > 99 | > 99 |
| SGMIB yield (purity (%)) | 30 | 73 | 91 | 96 | 99 | 99 |
| SGMIB (MBq) | 6.4 | 19.6 | 24.0 | 25.6 | 21.5 | 21.3 |
| Overall yield (%) | 27 | 67 | 83 | 88 | 91 | 90 |
| Collected volume (mL) | 2.1 | 2.0 | 2.1 | 2.1 | 2.2 | 2.1 |

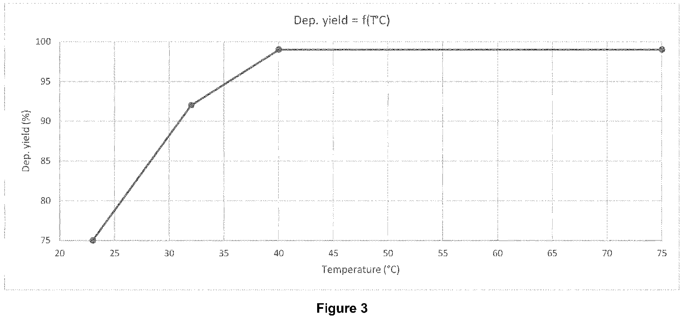

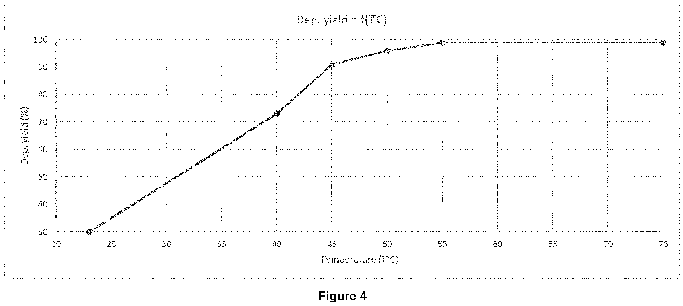

The evolution of deprotection yield according to heating (for 20 min) can be seen in Figure 3. The evolution of deprotection yield according to heating (for 20 min) can be seen in Figure 4.

A few reaction parameters are identified to influence the deprotection yield.

### Parameter 1. Temperature:

- Time Deprotection: 20 min
   Within a temperature range of 23°C- 75°C, deprotection reaction yield measures > 75%
   Within a temperature range of 32°C - 75°C, deprotection reaction yield measures > 92%
   Within a temperature range of 40°C - 75°C, deprotection reaction yield measures > 99%
- Time Deprotection: 5 min
   Within a temperature range of 23°C - 55°C, deprotection reaction yield measures > 30%
   Within a temperature range of 40°C - 75°C, deprotection reaction yield measures > 73%
   Within a temperature range of 55°C - 75°C, deprotection reaction yield measures > 99%

### Side products to (¹³¹I)SGMIB:

Deprotection reaction at 75°C during 20 min: side products to (¹³¹I)SGMIB range 3-12%
Deprotection reaction at 30-40°C during 20 min: side products to (¹³¹I)SGMIB < 1%

### Example 2: Automated reactions

The described experimental examples were obtained through a full automated procedure with an integrated cartridge heater using an ORA Neptis synthesizer. The experiments aimed at replicating the results obtained via manual syntheses.

The assessment of the efficacy of deprotection at different temperatures and reaction times have been performed by carrying-out HPLC analyses of the resulting product using identical chromatographic conditions compared to the manual syntheses.

The examples detailed below describe the performance of the novel method on the deprotection of Boc2-N-succinimidyl 4-guanidinomethyl-3-[(*)I]iodobenzoate ([*I]I-SGMIB-BOC₂), a radiohalogenation agent used for radiolabeling of targeting compounds.

### Materials and Methods

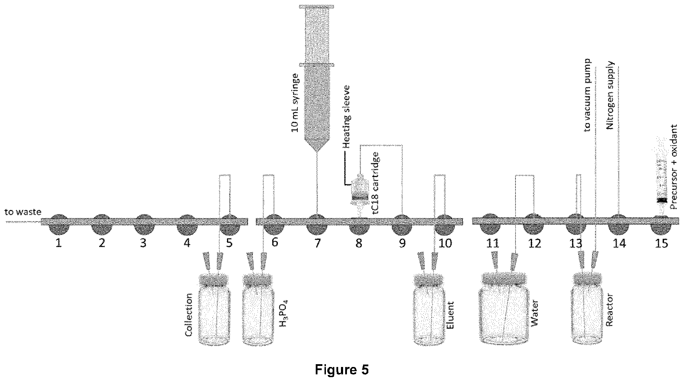

The manual synthesis was translated into a sequence that allows for use on the ORA Neptis synthesizer. This particular synthesizer for radiopharmaceuticals uses a disposable referred to as a "cassette", prepared prior to each experiment, and allows reproducing the manual synthesis in an automated fashion by performing a predefined sequence of "steps". Designing a suitable cassette and a sequence of steps allows for a reproducible radiochemical synthesis. The cassette layout used for the experimental examples is detailed below can be found in Figure 5.

The cassette consists in an ensemble of single-used manifolds comprising 3-way valves, on which are placed various consumables such as, non-exhaustively, vials filled with reagents, solid-phase extraction (SPE) or anion-exchange cartridges or syringes. Each step consists of an ensemble of setpoints (among those, for example, the position of a valve, the position of a syringe, the pressure and flow rate of nitrogen to be applied, the temperature of an oven or the actuation of the vacuum pump) that is applied for a pre-defined amount of time.

The following paragraph describes the full "template" sequence. The specific parameters that are varied between experimental examples are further detailed in Table 3. The results of the different experimental examples are described in Table 5.

Full automated sequence describing the different steps:
A. A solution containing the *radioactive isotope* is placed in the reactor (position 13) prior to radio-synthesis.
B. The solution is *dried* using a nitrogen flow and by heating the reactor.
C. Next, the *precursor* and the *oxidant* (position 15) are transferred into the reactor.
D. The *radiolabeling reaction* occurs in the reactor.
E. The reaction mixture is *diluted* with water (position 12) using the 10 mL syringe (position 7), and the resulting diluted solution is then loaded on the *tC18 cartridge* (position 8). The eluate resulting from loading the *tC18 cartridge* is sent to the waste.
F. The reactor and the *tC18 cartridge* are rinsed with water (position 12) using the 10 mL syringe (position 7).
G. The *acid* (position 6) is loaded on the *tC18 cartridge* using the 10 mL syringe (position 7). Once the *tC18 cartridge* has been saturated with acid, the valve in position 9 is closed, ensuring that the acid remains on the *tC18 cartridge* during deprotection.
H. The cartridge is heated for *a pre-defined amount of time* to allow for deprotection on the *tC18 cartridge.*
I. The *tC18 cartridge* is *rinsed* with water (position 12) using the 10 mL syringe (position 7), which forces the remaining *acid* and eluate to be sent to the waste.
J. The *eluent* for collection (position 10) is loaded on the *tC18 cartridge* using the 10 mL syringe (position 7). The eluate is *collected* in the collection vial (position 5).

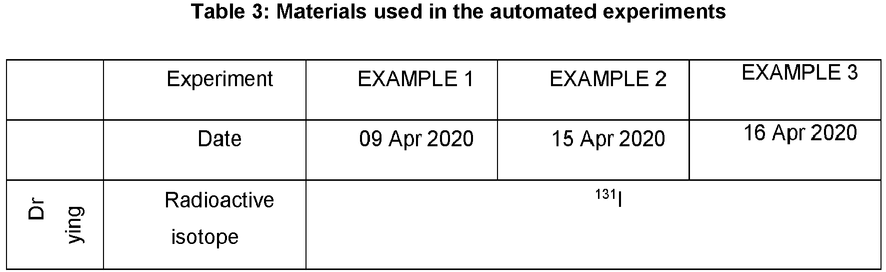

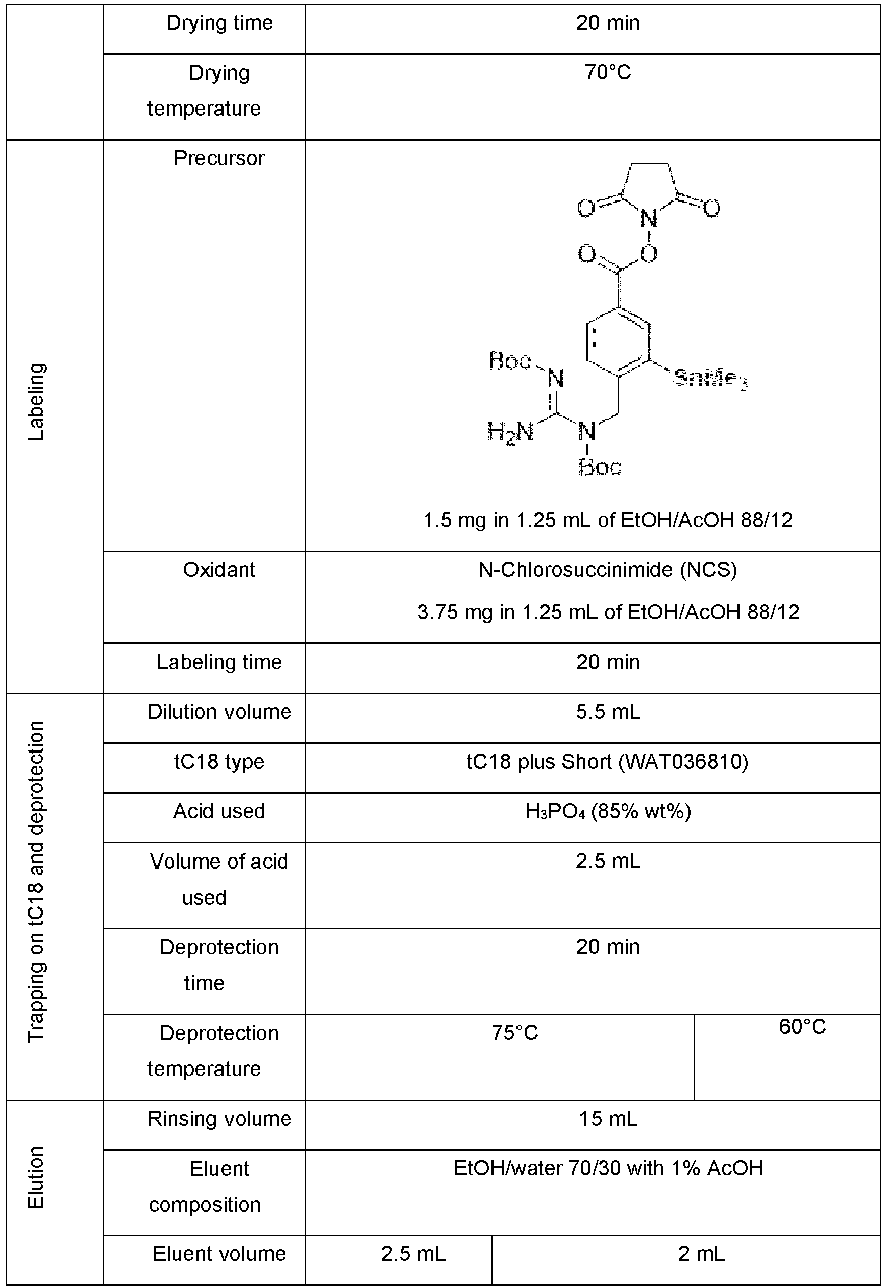

The full automated sequence described above allows for subsequent elution of pure [*I]I-SGMIB, constituted in EtOH/water 70/30 with 1% AcOH, into a conjugation vial that contains an appropriate amount of targeting compound in a conjugation buffer. After conjugation, [*I]I-SGMIB-labelled targeting compound is purified using a cartridge and eluted into formulation buffer ready for end-use. This part of the process is currently being translated into the synthesis sequence. An example of the cassette used for the automation of heated deprotection of BOC₂-protected [*I]I-SGMIB can be found in Figure 5.

### Chromatographic conditions

UV/Radio-HPLC analyses were performed on a Shimadzu LC-20AT Liquid chromatography system equipped with a C-18 column (X-Select, CSH, C18, 3.5 µ, 100 x 4.6 mm) with the flow rate set at 1.50 mL/min with the following gradient: t=0: 90 % A, 10 % B ; t=15 min: 100 % B ; with A = H₂O with 0.05 % TFA and B = ACN with 0.0 5% TFA.

### Detailed HPLC features:

- **HPLC:** Shimadzu LC-20AT - Prominence Liquid Chromatography 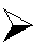 Gradient and solvent
- **UV Detector:** Shimadzu SPD-20A - Prominence UV/VIS Detector 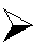 Dual detection: 220 nm / 254 nm
- **Radio Detector:** Elysia RAYTEST Sockel 3" GABI Nova 1.0 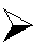 RA-detection: 5 µL loop
- **HPLC column:** X-Select, CSH, C18, 3.5 µ, 100 x 4.6 mm
- **Rheodyne injector:** 100 µL PEEK-loop
- **Software:** GINA X station Gabi Nova 31038

Each sample wass injected into an identical solvent mixture to the initial HPLC run conditions

H₂O/ACN + 0.05 % TFA (90/10)

### Reagents / cold references products: UV chromatograms (220-254 nm)

**Table 4: Elution times**

| **Labeling step** | | **Deprotection step** | |
|---|---|---|---|
| SGMTB-Boc₂ | 12.7 min | [*I]I-SGMIB- | 6.6 - 11.5 min |
| *N*-chlorosuccinimide (NCS) | 1.5 min | | |
| Acetic acid (HOAc) | 1.0 min | | 4.4 min |
| [*I]I-SGMIB-Boc₂ | 6.6 - 11.5 min | [*1]1-SGMIB | |

### Results

The experimental results are displayed in Table 5.

**Table 5: Experimental results for the automated reactions**

| Experiment | EXAMPLE 1 | EXAMPLE 2 | EXAMPLE 3 |
|---|---|---|---|
| Starting activity | 61.0 MBq | 33.4 MBq | 28.2 MBq |
| Activity collected at the end of the synthesis | 45.8 MBq | 24.1 MBq | 24.3 MBq |
| Collected activity | 75.08% | 72.16% | 86.17% |
| Deprotection efficacy | >99% | >99% | >99% |
| [*I]I-SGMIB yield (purity) | 94% | 94% | 97% |
| Overall yield | 70.6% | 67.8% | 83.6% |
| Collected volume after elution | 2.30 mL | 1.74 mL | 1.57 mL |

These first results indicate that the deprotection reaction yield measures > 99%, after reactions at both 75 and 60°C, and after only 20 minutes of incubation.

With this new invention, efficient heated cartridge deprotection was also confirmed for [*I]SGMIB that was radioiodinated in a reaction mixture consisting of either EtOH/HOAc or ACN/HOAc. These experiments indicated that the observed deprotection efficiency was independent of the constitution of the radioiodination reaction mixture (which takes place before deprotection). In addition, RP-HPLC results indicate that when the deprotection reaction takes place at 75°C, an impurity to [*I]SGMIB is observed, ranging from 3 - 12%. Deprotection reactions at 40°C do not give rise to this impurity (< 1%).

These findings are exceptional and contrast with the historical method for deprotection (where only about 30% corresponds to deprotected [*I]I-SGMIB after radioiodination in EtOH/HOAc. In addition, the level of impurities was much higher in the latter case, ranging about 25% in contrast to below 1% in the case of a method according to the invention.

### Example 3: Example device according to the invention

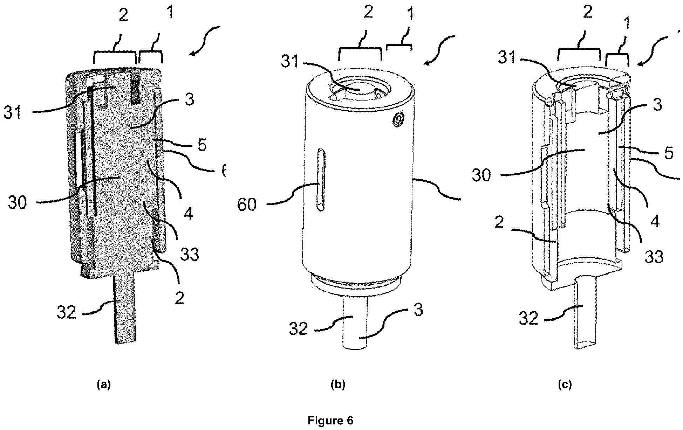

Figure 6 shows different views of an example embodiment of a device 1 and system 10 according to the invention. Figure 6a shows a first cut away view. Figure 6b shows a perspective view. Figure 6c shows a second cut away view.

The device 1 is adapted for receiving and heating a chemical reaction vessel 3 comprising a mixture (not shown). As illustrated in Figure 6, the system 10 comprises the device 1 and the chemical reaction vessel 3 that are mutually adapted in diameter and length such that the device 1 surrounds the chemical reaction vessel 3 in such a way that heat generated within a heating means 5 comprised in the device 1 may be carried over to the chemical reaction vessel 3. To this end, the device 1 comprises an opening 2 configured for receiving said reaction vessel 3, and the heating means 5 at least partially surrounding said opening. In embodiments as shown in the Figure, the chemical reaction vessel 3 is a cartridge. In embodiments, the cartridge is an SPE cartridge. The chemical reaction vessel 3 comprises a reactor chamber 30, an inlet 31, a housing 33, and an outlet 32.

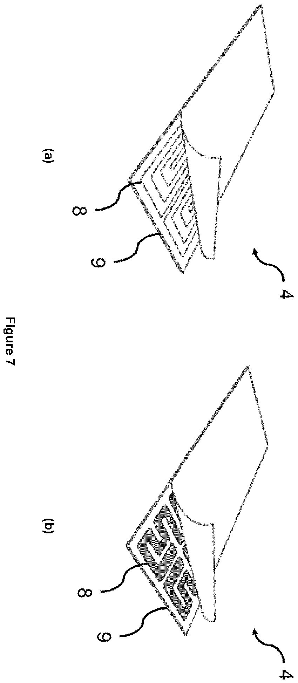

The heating means 5, which may, e.g., be according to embodiments of the heating means according to Example 2 and Figure 7, comprises an insulator polymer 9 and a resistive conductor 8 embedded in said insulator polymer 9.

The device 1 is configured for, when a chemical reaction vessel 3 comprising a mixture is present in said opening 2, heating the mixture present in said chemical reaction vessel according to a predetermined temperature requirement by powering said heating means 5.

The device 1 comprises a metal sleeve 4 placed between the chemical reaction vessel 3 and the heating means 5 and surrounding the chemical reaction vessel. In embodiments, the metal is copper.

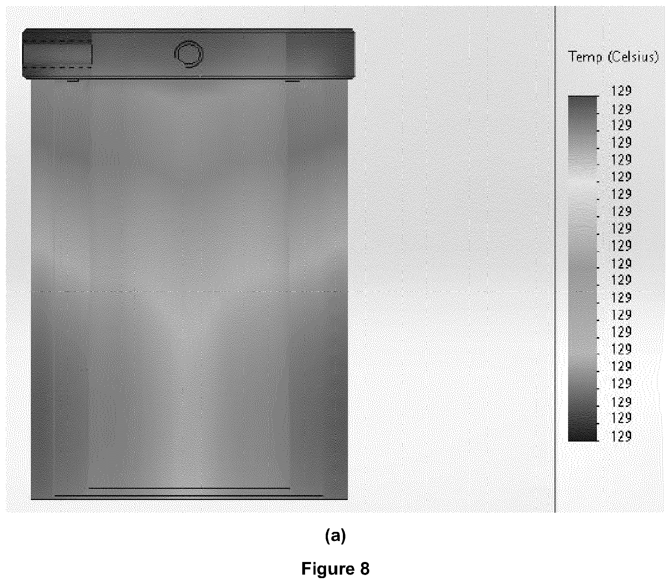

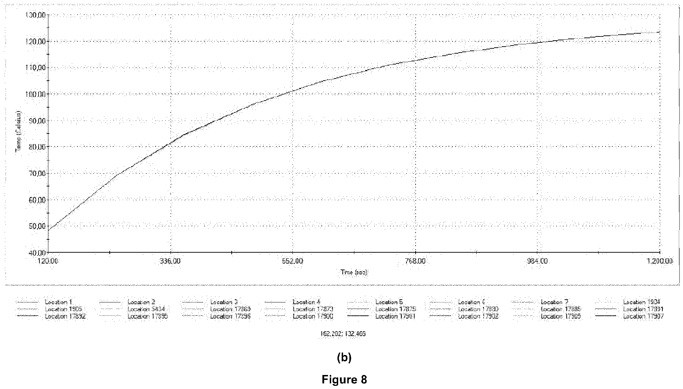

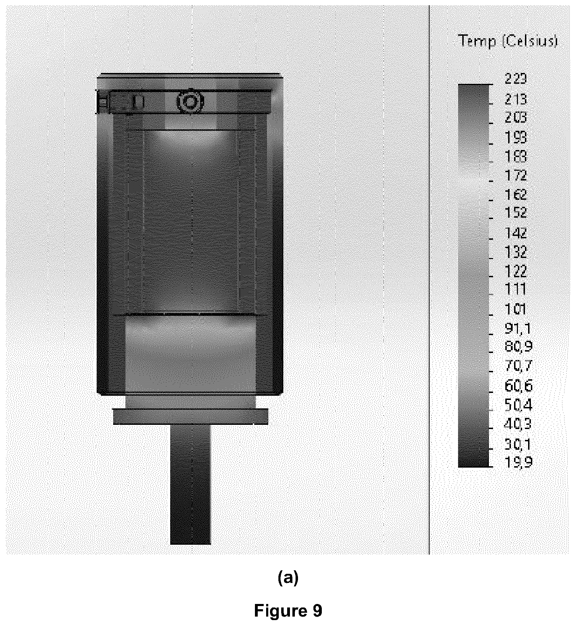

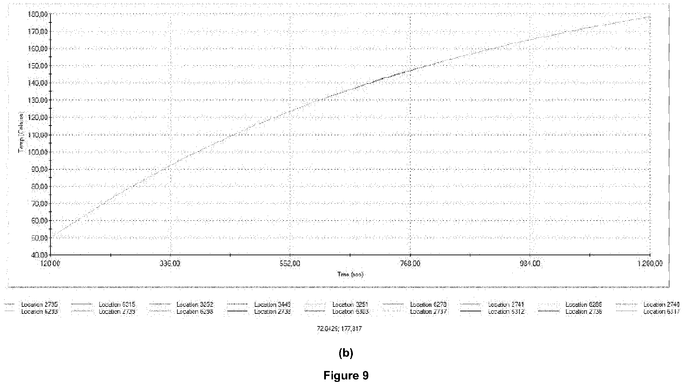

The use of a copper metal sleeve is illustrated in Figures 8 and 9 which show the simulated effect of power the heating means.

The heating means 5 comprises an adhesive layer for attaching said heating means 5 to the metal sleeve 4. This has the advantage of easier mounting of the device and/or improved structural integrity of the device and/or improved thermal conductivity for heat propagating from the heating means to the metal sleeve. The device 1 furthermore comprises a cover 6. The cover 6 conveniently keeps the device together. In embodiments according to this example, it comprises one or more holes 60 for the power supply to the heating pad 3.

In embodiments, the device 1 comprises a control unit and a temperature sensor, wherein said control unit is configured to maintain said predetermined temperature requirement relating to a temperature of said mixture being in a predetermined subrange comprised in a range from 30°C to 150°C, preferably from 30°C to 80°C, more preferably from 30°C to 50°C, by controlling the power supplied to said heating means 5 based on measurement by said temperature sensor. In preferred embodiment, said predetermined temperature requirement relates to reaching a stable temperature quickly and after a short equilibration time, preferably without any overshoot.

In embodiments as illustrated by Figure 6, the device 1 is a tubular sleeve and the opening 2 is a lumen surrounded and defined by said device. In embodiments, the heating means has a maximum dimension from 10 mm up to 200 mm, preferably from 20 mm up to 100 mm. Thereby, the length and/or resistance value and/or maximum power of the resistive conductor is adapted to the dimensions and/or requirements of the chemical reaction vessel chosen for use with the device and/or in function of the predetermined temperature requirement.

The chemical reaction vessel 3 further comprises a solid phase (not shown) suitable for acting as a facilitator in the chemical reaction. In embodiments, the chemical reaction vessel 3 comprises a metal, and/or a metal alloy and/or glass and/or fiber glass and/or an organic polymer. In preferred embodiments according to this example, the chemical reaction vessel 3 consists of one or more organic polymers. In embodiments the solid phase is a silica, preferably one or more of Sep-Pak tC18, Step-Pak C18, Oasis HLB, Oasis MCX, Oasis MAX and Sephadex LH-20. In preferred embodiments according to this example, the solid phase is Sep-Pak tC18.

In embodiments, a mixture is introduced via said inlet 31 for letting a chemical reaction facilitated by a solid phase take place within said reaction chamber 30 by electrically powering the heating means 5, after which the mixture may be extracted via the outlet 32. In embodiments, the mixture comprises a radioactive compound, wherein said chemical reaction is a chemical reaction of the radioactive compound facilitated by a solid phase.

The predetermined temperature requirement may relate to requiring a target temperature of 50 °C in the chemical reaction vessel for a duration of three minutes while starting from an initial temperature that is, e.g., 30°C or 40°C or 50°C or 60°C or 70°C. The predetermined temperature requirement furthermore specifies the maximum time for the transition from 60°C to 50 °C is three minutes requirement that the overshoot remains below 1°C.

In this example, the temperature requirement is met by means of a control unit being a programmable logical controller (PLC) comprising a 24 V power supply. Since the power of the heating means is low in this example, heat is supplied directly by the controller. Temperature is measured by means of a temperature sensor comprising a thermocouple board of the PLC. The control unit is configured such that temperature is controlled according to a PID control. This relates to specific parameters configured to avoid temperature overshoot.

### Example 4: Example heating means according to the invention

Figure 7 shows example embodiments of a heat pad 5 as heating element according to the invention. Figure 7a shows first embodiments of a heat pad comprising a wire wound heating element. Figure 7b shows second embodiments of a heat pad comprising an etched foil heating element. Both embodiments may, e.g., be combined with Example 1 as being the heating means or one of the heating means of the devices 1 of Example 1.

Both embodiments relate to a heat pad 5 comprising an insulator polymer 9 and a resistive conductor 8. Hereby, the first embodiments provide a resistive conductor 8 comprising a wire wound heating element, whereas the second embodiments provide a resistive conductor 8 comprising an etched foil resistive conductor. In third embodiments (not shown) according to the invention, the resistive conductor 8 comprises both one or more wire wound heating elements and one or more etched foil resistive conductors. In embodiments, the resistive conductor has an insulation resistance of more than 100 KΩ, preferably more than 1 MΩ, preferably at least 10 MΩ. In embodiments, the power rating is from 0.1 W up to 10 W, preferably from 0.5 up to 4 W, more preferably about 1 W or 1.25 W or 1.50 W or 1.75 W or 2 W.

In embodiments, the insulator polymer 9 comprises silicone and/or polyimide. The insulator polymer 9 of the second embodiments of Figure 7b may be different from that of the first embodiments of Figure 7a but may also be the same. In embodiments the insulator polymer 9 comprises both silicone and polyimide. The insulator polymer 9 preferably consists of a silicone or a polyimide. In embodiments, the melting temperature of the insulator polymer is higher than 150°C, preferably higher than 200°C.

In example embodiments, the heat pad 5 is a flexible sheet having a rectangular shape. The thickness of the flexible sheet is from 0.5 mm up to 1.5 mm, in examples it is from 0.5 mm up to 1.0 mm. In example embodiments, the thickness is 0.7 mm. The flexible sheet comprises a reinforcement layer covering one side of said flexible sheet. The reinforcement layer preferably consists of glass and/or fiber glass.

In example embodiments, the heat pad comprises an adhesive layer for attaching the heat pad to further portions of the device. For the device of Example 1, this may be the metal sleeve 4 or a cover 6 surrounding the heating means.

Figure 7a illustrates embodiments of the heat pad 5 comprising an insulator polymer 9 and a wire wound heating element 8. Such element 8 may for instance be created by spiralling fine resistance wires around a fiberglass cord, followed by laying out the element 8 in a predetermined pattern over the insulator polymer 9. However, other ways of creating such elements as known to the skilled person may be considered. In example embodiments, the pattern relates to one or more spirals extending over portions of the insulator polymer 9.

Figure 7b illustrates embodiments of the heat pad 5 comprising an insulator polymer 98 and an etched foil element 6. In such embodiments, the element is preferably created by acid etching a pattern in nickel alloy resistance foil, the pattern being a circuit. However, other ways of creating such elements as known to the skilled person may be considered. In example embodiments, the pattern relates to a curbed path or spiral comprising a large number of turns, e.g. more than ten turns.

In embodiments, the pattern is such that the total length of the resistive conductor maximum exceeds a maximal dimension of the heating means by a factor of at least two, preferably by a factor of at least five, more preferably by a factor of about ten. In example embodiments, the heating means has a maximum dimension of 50 mm, with a size of, e.g. 10 mm x 50 mm or 25 mm x 50 mm or 40 mm x 50 mm. In other example embodiments, the heating means has a maximum dimension of 30 mm, with a size of, e.g. 5 mm x 30 mm or 15 mm x 30 mm or 20 mm x 30 mm. In embodiments, the heating means has a maximum dimension from 10 mm up to 200 mm, preferably from 20 mm up to 100 mm. In embodiments, the total length of the resistive conductor maximum exceeds a maximal dimension of the heating means by a factor of at least two, with a length of, e.g., more than 50 mm or 100 mm or 200 mm or 300 mm or 500 mm or 800 mm. In embodiments, the minimal dimension, i.e. the thickness of the heating means, is from 0.1 mm up to 3.0 mm, preferably from 0.5 mm up to 1 mm, preferably about 0.6 mm or 0.7 mm or 0.8 mm or 0.9 mm.

In example embodiments, including the first and second embodiments of this example, the convex hull of said pattern covers at least 50% of the surface of the insulator polymer 9, more preferably at least 80% of the surface of the insulator polymer 9. In embodiments, the one or more respective spirals or curbed paths, preferably one, two, more than two, four, or more than four in number, extend over respective portions of the insulator polymer 9 that are nonoverlapping for at least 50% of their respective surfaces, preferably at least 80%.

Electrical power is supplied to the heat pad via a conducting wire, attached to the heat pad 5 via a hole in the cover 6, causing the heating of the heating pad. When the heat pad is used in a device and/or system such as that of Example 1, heat is transferred via conduction to the chemical reaction vessel. The heating of the chemical reaction vessel via the supply of electrical power to the heat pad 5 is controlled by means of a control unit and a temperature sensor.

## Claims

1. A method for performing a chemical reaction of a radioactive compound comprised in a mixture, wherein said method comprises the following steps:
(a) contacting said mixture with a solid phase, followed by
(b) heating said mixture to a temperature selected in the range from 30°C up to 150°C,
wherein steps (a) and (b) do not involve contacting said solid phase with an alkaline solution,
wherein said chemical reaction does not result in the formation of a new bond on a radionuclide comprised in said radioactive compound, wherein said radioactive compound does not comprise fluorine-18.

2. A method according to claim 1, wherein (a) contacting said mixture with said solid phase results in the attachment of said radioactive compound to said solid phase, and wherein said method further comprises the step of (c) detaching said radioactive compound from said solid phase by contacting said solid phase with an eluent, , wherein said eluent is selected from the group consisting of aqueous solutions, organic solvents or a mixtures thereof, more preferably wherein said organic solvent is a mixture of water and ethanol, most preferably wherein said organic solvent is ethanol.

3. A method according to claim 1 or 2, wherein said chemical reaction comprises a hydrolysis of said radioactive compound, wherein said hydrolysis occurs during said heating, wherein said hydrolysis is an acid hydrolysis, preferably wherein said acid hydrolysis comprises the use of an acid selected from the group consisting of phosphoric acid, hydrochloric acid, sulfuric acid, trifluoroacetic acid and aqueous mixtures thereof, more preferably from the group consisting of phosphoric acid, hydrochloric acid, sulfuric acid, and aqueous mixtures thereof, most preferably wherein said acid is 80 wt% phosphoric acid.

4. A method according to claim 3, wherein said hydrolysis comprises a removal of a protecting group from said radioactive compound, preferably wherein said removal results in a deprotected radioactive compound, more preferably wherein said protecting group is selected from the group consisting of tert-butylcarbamate (t-Boc or Boc), tert-buylester (OtBu), Benzylester (BzO), benzylidene, tetrahydropyranyl ether (THP), acetal, trityl (Trt) and methoxymethyl ether (MOM), and most preferably wherein said protecting group is tert-butylcarbamate (t-Boc or Boc).

5. A method according to any one of claims 1 to 6, wherein said temperature is selected in the range from 30°C up to 70°C, preferably in the range from 30°C up to 55°C, more preferably wherein said temperature is selected in the range from 30°C up to 70°C and the duration of said heating is selected in the range from 1 minute up to 15 minutes, most preferably wherein said temperature is selected in the range from 30°C up to 55°C and the duration of said heating is selected in the range from 1 minute up to 10 minutes.

6. A method according to claim 4 or 5, wherein said method comprises the following step:
(d) attaching a biological moiety to the deprotected radioactive compound, preferably obtained at the end of step (c) as defined in claim 2, wherein said attaching results in a radiolabeled biological moiety, preferably wherein said biological moiety is a polymer of amino acids.

7. A method according to claim 6, wherein said biological moiety is an antibody or a fragment thereof, preferably wherein said antibody or fragment thereof is a diagnostic and/or a therapeutic compound, more preferably wherein said diagnostic and/or therapeutic compound is targeted against an antigen expressed in a cell, most preferably in a tumor cell, more preferably wherein said antigen is HER2.

8. A method according to claim 7, wherein said antibody or fragment thereof is a heavy chain variable domain derived from a heavy chain antibody (V_{HH}), or a fragment thereof, preferably wherein said heavy chain antibody (V_{HH}), or a fragment thereof, has at least 80% amino acid identity with or has an amino acid sequence SEQ ID NO: 7 or SEQ ID NO: 8.

9. A method according to any one of claims 1 to 8, wherein said radioactive compound comprises a radionuclide selected from the group consisting of α-emitters and, β-emitters, preferably selected from the list consisting of hydrogen-3, astatine-211, carbon-11, carbon-14, bromine-76, iodine-123, iodine-124, iodine-125, iodine-131, phosphorus-32, and sulfur-35, most preferably wherein said radionuclide is selected from the group consisting of astatine-211, iodine-123, iodine-124 and iodine-131.

10. A method according to any one of claims 1 to 9, wherein said radioactive compound is N-succinimidyl-4-(1,2-bis(tert-butoxycarbonyl)guanidino)methyl-3-[(131)I]iodobenzoate (Boc₂-[¹³¹I]SGMIB), preferably wherein N-succinimidyl-4-(1,2-bis(tert-butoxycarbonyl)guanidino)methyl-3-[(131)I]iodobenzoate (Boc₂-[¹³¹I]SGMIB) is converted to N-succinimidyl-4-guanidinomethyl-3-[(131)I]iodobenzoate ([¹³¹I]SGMIB) with a yield of at least 30% during heating, as determined by quantitative HPLC, wherein the duration of said heating is selected in the range from 1 minute to 10 minutes, more preferably from 1 minute up to 5 minutes.

11. A method according to any one of claims 1 to 10, wherein said method comprises the use of a device according to any one of claims 12 to 17 or a system according to any one of claims 18 to 20, wherein said (b) heating said mixture during said method is achieved by said powering of said heating means comprised in said device or said system.

12. A device (1) for receiving and heating a chemical reaction vessel (3) comprising a mixture, said device (1) comprising a heating means (5) and an opening (2) configured for receiving said chemical reaction vessel (3), said heating means (5) at least partially surrounding said opening;
wherein said heating means (5) comprises:
- an insulator polymer (9),
- a resistive conductor (8) embedded in said insulator polymer;
wherein said device (1) is configured for, when said chemical reaction vessel (3) comprising said mixture is present in said opening (2), heating the mixture present in said chemical reaction vessel according to a predetermined temperature requirement by powering said heating means (5),
preferably wherein said device (1) is a tubular sleeve and said opening is a lumen surrounded by said device (1).

13. Device (1) according to claim 12, wherein said insulator polymer (9) is a silicone or a polyimide, preferably wherein the melting temperature of said insulator polymer (9) is higher than 150°C, preferably higher than 200°C.

14. Device (1) according to claim 12 or 13, wherein said resistive conductor (8) is an etched foil heating element or a wire wound heating element.

15. Device (1) according to any one of claims 12 to 14, wherein said heating means (5) is a flexible sheet,
- preferably wherein said flexible sheet has a rectangular shape; and/or
- preferably wherein a thickness of said flexible sheet is from 0.5 mm up to 1.5 mm, more preferably from 0.5 mm up to 1.0 mm
- preferably wherein said flexible sheet comprises a reinforcement layer covering one side of said flexible sheet, more preferably wherein said reinforcement layer consists of glass and/or fiber glass.

16. Device (1) according to any one of claims 12 to 15, wherein said device comprises a metal sleeve (4) placed between the heating means and the opening and surrounding the opening, said metal preferably being copper.

17. Device (1) according to any one of claims 12 to 16, wherein said device (1) comprises a control unit and preferably further comprises a temperature sensor, wherein said control unit is configured to maintain said predetermined temperature requirement relating to a temperature of said mixture being in a predetermined subrange comprised in a range from 30°C to 150°C, preferably from 30°C to 80°C, more preferably from 30°C to 50°C, by controlling the power supplied to said heating means, preferably said controlling being based on measurement by said temperature sensor.

18. A system (10) for performing a chemical reaction in a mixture, comprising:
- a device (1) according to any one of claims 12 to 16;
- a chemical reaction vessel (3) placed within the opening (2) of device (1), said chemical reaction vessel (3) comprising a chamber (30), an inlet (31), and a solid phase preferably suitable for acting as a facilitator in said chemical reaction;
wherein said system (10) is configured for, when said mixture is inserted in the chamber (30) through said inlet (31), heating said mixture present in said chemical reaction vessel (3) according to a predetermined temperature requirement by powering said heating means (5), thereby allowing said chemical reaction to take place within said chamber (30).

19. System (10) according to claim 18, wherein said chemical reaction vessel (3) further comprises an outlet (32), preferably wherein said chemical reaction vessel is an SPE cartridge.

20. A method according to any one of claims 1 to 11, or a device according to any one of claims 12 to 17, or a system according to any one of claims 18 to 20, wherein said solid phase used in step (a) of said method or said solid phase comprised in said device or said method is a silica, preferably selected from the group consisting of Sep-Pak tC18, Step-Pak C18, Oasis HLB, Oasis MCX, Oasis MAX, Sephadex LH-20 and combinations thereof, more preferably Sep-Pak tC18.


SEQUENCE LISTING

<110>  Camel-IDS NV/SA

<120>  Solid-phase supported reactions of radioactive compounds

<130>  P6095055EP

<160>  13

<170>  PatentIn version 3.5

<210>  1
<211>  8
<212>  PRT
<213>  Artificial Sequence

<220>
<223>  CDR1 sequence of VHH 2Rs15d

<400>  1

Gly Tyr Ile Phe Asn Ser Cys Gly
1               5


<210>  2
<211>  7
<212>  PRT
<213>  Artificial Sequence

<220>
<223>  CDR2 sequence of VHH 2Rs15d

<400>  2

Ile Ser Gly Asp Gly Asp Thr
1               5


<210>  3
<211>  9
<212>  PRT
<213>  Artificial Sequence

<220>
<223>  CDR3 sequence of VHH 2Rs15d

<400>  3

Ala Val Cys Tyr Asn Leu Glu Thr Tyr
1               5


<210>  4
<211>  8
<212>  PRT
<213>  Artificial Sequence

<220>
<223>  CDR1 sequence of VHH 2Rb17c

<400>  4

Gly Phe Ile Phe Ser Asn Asp Ala
1               5

<210>  5
<211>  8
<212>  PRT
<213>  Artificial Sequence

<220>
<223>  CDR2 sequence of VHH 2Rb17c

<400>  5

Ile Asn Trp Ser Gly Thr His Thr
1               5

<210>  6
<211>  10
<212>  PRT
<213>  Artificial Sequence

<220>
<223>  CDR3 sequence of VHH 2Rb17c

<400>  6

Val Thr Gly Tyr Gly Val Thr Lys Thr Pro
1               5                   10

<210>  7
<211>  115
<212>  PRT
<213>  Artificial Sequence

<220>
<223>  VHH sequence 2Rs15d

<400>  7

Gln Val Gln Leu Gln Glu Ser Gly Gly Gly Ser Val Gln Ala Gly Gly
1               5                   10                  15

Ser Leu Lys Leu Thr Cys Ala Ala Ser Gly Tyr Ile Phe Asn Ser Cys
            20                  25                  30

Gly Met Gly Trp Tyr Arg Gln Ser Pro Gly Arg Glu Arg Glu Leu Val
        35                  40                  45

Ser Arg Ile Ser Gly Asp Gly Asp Thr Trp His Lys Glu Ser Val Lys
    50                  55                  60

Gly Arg Phe Thr Ile Ser Gln Asp Asn Val Lys Lys Thr Leu Tyr Leu
65                  70                  75                  80

Gln Met Asn Ser Leu Lys Pro Glu Asp Thr Ala Val Tyr Phe Cys Ala
                85                  90                  95

Val Cys Tyr Asn Leu Glu Thr Tyr Trp Gly Gln Gly Thr Gln Val Thr
            100                 105                 110

Val Ser Ser
        115

<210>  8
<211>  117
<212>  PRT
<213>  Artificial Sequence

<220>
<223>  VHH sequence 2Rb17c

<400>  8

Gln Val Gln Leu Gln Glu Ser Gly Gly Gly Leu Val Gln Pro Gly Gly
1               5                   10                  15

Ser Leu Arg Leu Ser Cys Ala Ala Ser Gly Phe Ile Phe Ser Asn Asp
            20                  25                  30

Ala Met Thr Trp Val Arg Gln Ala Pro Gly Lys Gly Leu Glu Trp Val
        35                  40                  45

Ser Ser Ile Asn Trp Ser Gly Thr His Thr Asn Tyr Ala Asp Ser Val
    50                  55                  60

Lys Gly Arg Phe Thr Ile Ser Arg Asp Asn Ala Lys Arg Thr Leu Tyr
65                  70                  75                  80

Leu Gln Met Asn Ser Leu Lys Asp Glu Asp Thr Ala Leu Tyr Tyr Cys
                85                  90                  95

Val Thr Gly Tyr Gly Val Thr Lys Thr Pro Thr Gly Gln Gly Thr Gln
            100                 105                 110

Val Thr Val Ser Ser
        115

<210>  9
<211>  127
<212>  PRT
<213>  Artificial Sequence

<400>  9

Asp Val Gln Leu Val Glu Ser Gly Gly Gly Ser Val Gln Gly Ala Ala
1               5                   10                  15

Gly Gly Ser Leu Arg Leu Ser Cys Ala Ala Ser Asp Ile Thr Tyr Ser
            20                  25                  30

Thr Asp Cys Met Gly Trp Phe Arg Gln Ala Pro Gly Lys Glu Arg Glu
        35                  40                  45

Gly Val Ala Thr Ile Asn Asn Gly Arg Ala Ile Thr Tyr Tyr Ala Asp
    50                  55                  60

Ser Val Lys Gly Arg Phe Thr Ile Ser Gln Asp Asn Ala Lys Asn Thr
65                  70                  75                  80

Val Tyr Leu Gln Met Asn Ser Leu Arg Pro Lys Asp Thr Ala Ile Tyr
                85                  90                  95

Tyr Cys Ala Ala Arg Leu Arg Ala Gly Tyr Cys Tyr Pro Ala Asp Tyr
            100                 105                 110

Ser Met Asp Tyr Trp Gly Lys Gly Thr Gln Val Thr Val Ser Ser
        115                 120                 125

<210>  10
<211>  127
<212>  PRT
<213>  Artificial Sequence

<400>  10

Asp Val Gln Leu Glu Glu Ser Gly Gly Gly Ser Val Gln Ala Gly Gly
1               5                   10                  15

Ser Leu Arg Leu Ser Cys Ala Ala Ser Gly Tyr Ile Tyr Ser Thr Tyr
            20                  25                  30

Cys Met Gly Trp Phe Arg Gln Ala Pro Gly Lys Glu Arg Glu Gly Val
        35                  40                  45

Ala Ala Ile Asn Asp Val Gly Gly Ser Val Tyr Tyr Ala Asp Ser Val
    50                  55                  60

Lys Gly Arg Phe Thr Ile Ser Gln Asp Ile Ala Gln Asp Thr Met Tyr
65                  70                  75                  80

Leu Gln Met Asn Asp Leu Thr Pro Glu Asn Thr Val Thr Tyr Thr Cys
                85                  90                  95

Ala Ala Leu Arg Cys Leu Ser Asp Ser Asp Pro Asp Thr Arg Val His
            100                 105                 110

Met Tyr Tyr Asp Trp Gly Gln Gly Thr Gln Val Thr Val Ser Ser
        115                 120                 125

<210>  11
<211>  125
<212>  PRT
<213>  Artificial Sequence

<400>  11

Asp Val Gln Leu Glu Glu Ser Gly Gly Gly Ser Val Gln Thr Gly Gly
1               5                   10                  15

Ser Leu Arg Leu Ser Cys Ala Ala Ser Gly Tyr Thr Tyr Ser Ser Ala
            20                  25                  30

Cys Met Gly Trp Phe Arg Gln Gly Pro Gly Lys Glu Arg Glu Ala Val
        35                  40                  45

Ala Asp Val Asn Thr Gly Gly Arg Arg Thr Tyr Tyr Ala Asp Ser Val
    50                  55                  60

Lys Gly Arg Phe Thr Ile Ser Gln Asp Asn Thr Lys Asp Met Arg Tyr
65                  70                  75                  80

Leu Gln Met Asn Asn Leu Lys Pro Glu Asp Thr Ala Thr Tyr Tyr Cys
                85                  90                  95

Ala Thr Gly Pro Arg Arg Arg Asp Tyr Gly Leu Gly Pro Cys Asp Tyr
            100                 105                 110

Asn Tyr Trp Gly Gln Gly Thr Gln Val Thr Val Ser Ser
        115                 120                 125

<210>  12
<211>  112
<212>  PRT
<213>  Artificial Sequence

<400>  12

Glu Val Gln Leu Glu Glu Ser Gly Gly Gly Leu Val Gln Pro Gly Gly
1               5                   10                  15

Ser Leu Thr Leu Ser Cys Ala Ala Ser Gly Tyr Thr Phe Thr Asn Cys
            20                  25                  30

Ala Ala Gly Trp Tyr Arg Gln Ala Pro Gly Lys Glu Cys Glu Leu Val
        35                  40                  45

Ala Ser Ile Phe Ser Gly Asn Arg Thr Asn Tyr Ala Asp Ser Val Lys
    50                  55                  60

Gly Arg Phe Thr Ile Ser Arg Asp Asn Thr Lys Asp Ile Val Tyr Leu
65                  70                  75                  80

Gln Met Asn Ser Leu Lys Pro Glu Asp Thr Thr Val Tyr Tyr Cys Asp
                85                  90                  95

Ala Arg Thr Pro Cys Trp Gly Gln Gly Thr Gln Val Thr Val Ser Ser
            100                 105                 110

<210>  13
<211>  121
<212>  PRT
<213>  Artificial Sequence

<400>  13

Glu Val Gln Leu Glu Glu Ser Gly Gly Gly Ser Val Gln Ala Gly Gly
1               5                   10                  15

Ser Leu Arg Leu Ser Cys Ala Ala Ser Gly Tyr Thr Phe Leu Gln Leu
            20                  25                  30

Leu His Gly Trp Phe Arg Gln Ala Pro Gly Lys Glu Arg Glu Val Val
        35                  40                  45

Ala Arg Phe Asn Thr Asp Ile Asn Lys Thr Phe Tyr Leu Glu Ser Val
    50                  55                  60

Lys Gly Arg Phe Thr Leu Ser Gln Asp Asn Ala Lys Asn Thr Leu Tyr
65                  70                  75                  80

Leu Gln Met Asn Ser Leu Lys Pro Glu Asp Thr Ala Ile Tyr Tyr Cys
                85                  90                  95

Ala Ala Ser Arg Pro Asp Ser Thr Cys Asp Tyr Phe Ala Tyr Arg Gly
            100                 105                 110

Gln Gly Thr Gln Val Thr Val Ser Ser
        115                 120